# EUROPEAN PATENT APPLICATION

(11) **EP 4 190 911 A1**
(43) Date of publication of application: **07.06.2023**
(21) Application number: 21857775.7
(22) Date of filing: 20.08.2021
(51) Int. Cl.: C12Q 1/68

(54) **METHOD AND DRUG FOR TUMOR TREATMENT**

(30) Priority: 20.08.2020 WO PCT/CN2020/110298
(71) Applicant: Talengen International Limited, Hong Kong 999077 (HK)
(72) Inventor: LI, Jinan, Beijing 100089 (CN)
(74) Representative: karo IP
(86) International application number: PCT/CN2021/113850
(87) International publication number: WO 2022/037687

(57) **Abstract**

The present invention provides a method for treating a tumor in a subject, maintaining or improving body organ function under tumor conditions, and prolonging the survival period of the subject, comprising administering to the subject a prophylactically and/or therapeutically effective amount of components such as plasminogen of a plasminogen activation pathway. The present invention also provides a medicament comprising plasminogen, a pharmaceutical composition, a formulation and a kit for treating a tumor in a subject.

## Description

### TECHNICAL FIELD

The present application relates to the effect of a component of the plasminogen activation pathway (e.g., plasminogen) in preventing and treating a tumor, thereby providing a new therapeutic strategy for preventing and treating a tumor.

### BACKGROUND ART

Malignant tumor is a disease that seriously threatens human health. According to statistics from the American Cancer Society, cancer is the leading cause of death in developed countries, accounting for 21.6% of the deaths. According to WHO statistics, in the past few years, the number of patients who died of cancer in the world has reached more than 7 million every year, it is very close to the number of cardiovascular diseases, and is expected to exceed the number of cardiovascular diseases to become the world's leading cause of death. The incidence of tumor remains high with an upward trend, and it has seriously threatened life and health. However, the pathogenesis of tumors is complicated, the effect of tumor treatment is poor, the rate of recurrence and metastasis is high, and the side effects of tumor treatment are large. Therefore, the research on prevention and treatment of tumors is also a difficult point. The present application finds that the component of the plasminogen activation pathway (e.g., plasminogen) can significantly inhibit the growth of tumors, and it may become a new approach for the prevention and treatment of tumors.

### SUMMARY OF THE APPLICATION

In one aspect, the present application relates to a method for treating a tumor, comprising administrating to a subject suffering from a tumor an effective amount of one or more compounds selected from the group consisting of: a component of plasminogen activation pathway, a compound directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of a fibrinolysis inhibitor.

In one aspect, the present application also relates to the use of one or more compounds selected from the following in the preparation of a medicament for treating a tumor: a component of plasminogen activation pathway, a compound directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of a fibrinolysis inhibitor.

In one aspect, the present application also relates to the use of one or more compounds selected from the following or a pharmaceutical composition comprising one or more compounds selected from the following in the treatment of a tumor: a component of plasminogen activation pathway, a compound directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of a fibrinolysis inhibitor.

In some embodiments, the component of plasminogen activation pathway is selected from the group consisting of: plasminogen, recombinant human plasmin, Lys-plasminogen, Glu-plasminogen, plasmin, a variant of plasminogen and plasmin and an analog thereof comprising one or more kringle domains and protease domains of plasminogen and plasmin, mini-plasminogen, mini-plasmin, micro-plasminogen, micro-plasmin, delta-plasminogen, delta-plasmin, an activator of plasminogen, tPA and uPA.

In some embodiments, the antagonist of fibrinolysis inhibitor is PAI-1, an inhibitor of complement C1, or an inhibitor of α2 anti-plasmin or α2 macroglobulin, e.g., an antibody.

In some embodiments, the compound is plasminogen.

In some embodiments, the present application provides a method for treating a tumor, comprising administering an effective amount of plasminogen to a subject suffering from a tumor.

In some embodiments, the tumor is a malignant tumor. In some embodiments, the tumor is a cancer. In some embodiments, the tumor is a solid tumor. In some embodiments, the tumor is a tumor of the digestive system or the respiratory system. In some embodiments, the tumor is any one or more selected from the group consisting of: oral cancer, esophageal cancer, gastric cancer, small intestine cancer, colon cancer, rectal cancer, lung cancer, liver cancer, hepatocellular carcinoma, pancreatic cancer, gallbladder cancer, non-small cell lung (NSCL) cancer, bronchoalveolar cell lung cancer, breast cancer, ovarian cancer, cervical cancer, fallopian tube cancer, endometrial cancer, vaginal cancer, prostate cancer, urethral cancer, penile cancer, kidney cancer, ureter cancer, renal cell cancer, renal pelvis cancer, bladder cancer, head and neck cancer, skin cancer, melanoma, mesothelioma, bone cancer, thyroid cancer, parathyroid cancer, adrenal cancer, soft tissue sarcoma, glioma, pleomorphic glioblastoma, astrocytoma, Schwann cell tumor, ependymoma, medulloblastoma, meningioma, squamous cell carcinoma, and pituitary adenoma.

In some embodiments, the plasminogen of the present application has one or more effects selected from the group consisting of: reducing tumor volume, improving general survival of a subject suffering from a tumor, delaying tumor progression, inhibiting the growth of tumor cells, improving the survival rate, prolonging the survival period of a subject suffering from a tumor, relieving cancer pain, inhibiting tumor angiogenesis, promoting tumor cell necrosis or apoptosis, promoting anti-tumor immune response, regulating the expression of tumor-associated antigens or the surface molecules of lymphocytes, reducing the damage of tissues and organs caused by cancer cells, and promoting the recovery of tumor-damaged tissue structures or functions.

In some embodiments, the plasminogen level in blood or the plasminogen level in tumor tissue or non-tumor tissue of a subject suffering from a tumor is higher than, equal to or lower than the plasminogen level in blood or the plasminogen level in corresponding non-tumor tissue of a healthy subject. In some embodiments, the fibrin level in blood or the fibrin level in tumor tissue or non-tumor tissue of a subject suffering from a tumor is higher than, equal to or lower than the fibrin level in blood or the fibrin level in corresponding non-tumor tissue of a healthy subject.

In some embodiments, the plasminogen of the present application is administered in combination with one or more selected from the group consisting of: chemotherapy, radiotherapy, surgical therapy, cell therapy and immunotherapy. In some embodiments, the chemotherapeutic agents include, for example, alkylating agents, antimetabolites (e.g., folic acid analogs), pyrimidine analogs, purine analogs, antimitotic agents, antibiotics, cytokines, platinum coordination complexes, substituted urea, hormones, adrenal corticosteroid antagonists, anti-estrogens, anti-androgens, etc.

In some embodiments, the plasminogen is selected from the group consisting of:
Glu-plasminogen, Lys-plasminogen, mini-plasminogen, micro-plasminogen, delta-plasminogen, or a conservatively substituted variant thereof. In some embodiments, the plasminogen is a plasminogen protein comprising a serine protease domain and/or a lysine binding domain. In some embodiments, the plasminogen comprises a plasminogen protein having at least 75%, 80%, 85%, 90%, 95%, 96%, 97%, 98% or 99% sequence identity with SEQ ID NO: 14 and having proteolytic activity. In some embodiments, the plasminogen has at least 75%, 80%, 85%, 90%, 95%, 96%, 97%, 98% or 99% sequence identity with SEQ ID NO: 2, 6, 8, 10 or 12, and still has plasminogen activity.

In some embodiments, the plasminogen is selected from the group consisting of: Glu-plasminogen, Lys-plasminogen, mini-plasminogen, micro-plasminogen, delta-plasminogen, or a variant thereof retaining plasminogen activity. In some embodiments, the plasminogen is a protein with addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid based on SEQ ID NO: 2, 6, 8, 10 or 12, and still has plasminogen activity.

In some embodiments, the plasminogen is natural or synthetic human plasminogen, or a variant or fragment thereof still retaining plasminogen activity. In some embodiments, the plasminogen is a human plasminogen ortholog from a primate or rodent, or a variant or fragment thereof still retaining plasminogen activity. In some embodiments, the amino acid sequence of the plasminogen is represented by SEQ ID NO: 2, 6, 8, 10 or 12. In some embodiments, the plasminogen is human natural plasminogen.

In some embodiments, the subject is a human. In some embodiments, the subject is low, or deficient or lacking in plasminogen. In some embodiments, the low level, or lack or deficiency of plasminogen is congenital, secondary and/or local.

In some embodiments, the pharmaceutical composition comprises a pharmaceutically acceptable carrier and plasminogen for use in the above methods. In some embodiments, the kit may be a prophylactic or therapeutic kit, comprising: (i) plasminogen for use in the above methods, and (ii) means for delivering the plasminogen to the subject. In some embodiments, the means is a syringe or vial. In some embodiments, the kit further comprises a label or instructions for instructing the administration of the plasminogen to the subject to perform any of the above methods.

In some embodiments, the article of manufacture comprises: a container comprising a label; and further comprises (i) plasminogen or a pharmaceutical composition comprising the plasminogen for use in the above method, wherein the label instructs the administration of the plasminogen or composition to the subject to perform any of the above methods.

In some embodiments, the kit or article of manufacture further comprises one or more additional means or containers containing other medicaments. In some embodiments, the other medicament is an antineoplastic medicament.

In some embodiments of the above methods, the plasminogen is administrated by systemic or topical administration for therapy, preferably by intravenous, intramuscular, or subcutaneous administration of plasminogen. In some embodiments of the above methods, the plasminogen is administrated in combination with a suitable polypeptide carrier or stabilizer. In some embodiments of the above methods, the plasminogen is administrated per day at the amount of 0.0001-2000 mg/kg, 0.001-800 mg/kg, 0.01-600 mg/kg, 0.1-400 mg/kg, 1-200 mg/kg, 1-100 mg/kg, or 10-100 mg/kg (by per kilogram of body weight); or 0.0001-2000 mg/cm², 0.001-800 mg/cm², 0.01-600 mg/cm², 0.1-400 mg/cm², 1-200 mg/cm², 1-100 mg/cm², or 10-100 mg/cm² (by per square centimeter of body surface area), preferably repeating at least once, and preferably administrating at least daily.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

### BRIEF DESCRIPTION OF THE DRAWINGS

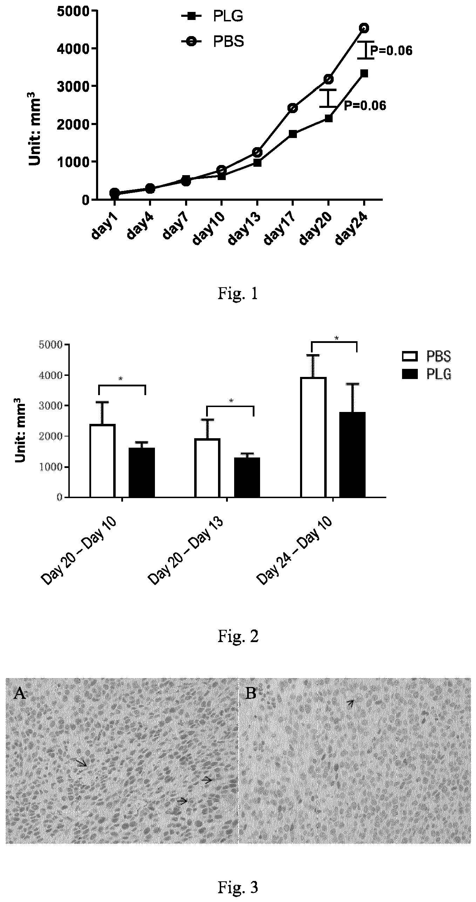

Fig. 1 shows the measurement results of tumor volumes of the colon cancer model mice after administrating plasminogen by tail vein for 23 days. The results show that the growth rate of colon tumors in the plasminogen group is significantly lower than that of the vehicle PBS control group during the administration period; and on Day 20 and Day 24, the statistical differences in tumor size between the two groups are close to significant (P=0.06); particularly, the tumor volume in the plasminogen group is reduced by 40% on Day 17, by 26% on Day 20, and by 32% on Day 24 as compared with the PBS control group.
Fig. 2 shows the calculation results of tumor volume changes of the colon cancer model mice after administrating plasminogen by tail vein. The differences between the tumor volume on Day 20 and the tumor volumes on Day 10 and Day 13, and the differences between the tumor volume on Day 24 and the tumor volume on Day 10 are calculated; and the results show that the tumor volume increase in the plasminogen group is significantly smaller than that in the vehicle PBS control group, and the statistical difference is significant (* indicates P<0.05).
Figs. 3A-3B show the representative images of CD31 immunohistochemical staining of tumor mass of the colon cancer model mice after administrating plasminogen by tail vein for 29 days. Fig. 3A is the vehicle PBS control group, and Fig. 3B is the plasminogen group. The results show that the expression of CD31 (marked by the arrow) in the tumor tissues of the plasminogen group is significantly less than that of the vehicle PBS control group. It shows that plasminogen can inhibit the expression of vascular endothelial cell marker CD31 in tumor tissue, indicating that plasminogen can inhibit the formation of new blood vessels in colon cancer tumor tissue.

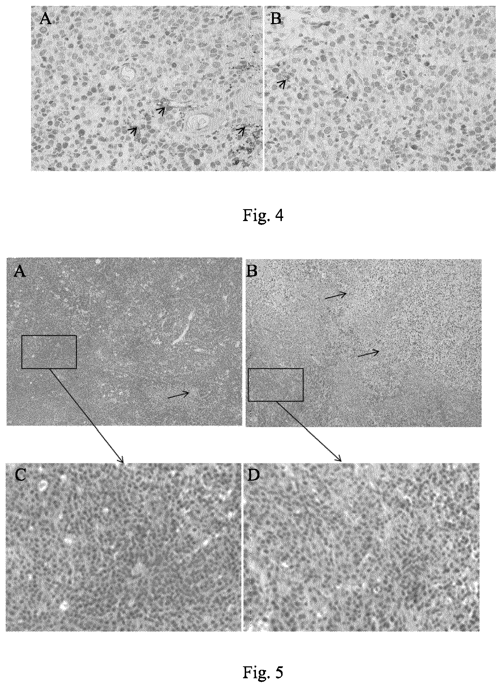

Figs. 4A-4B show the representative images of LYVE-1 immunohistochemical staining of tumor tissue of the colon cancer model mice after administrating plasminogen by tail vein for 29 days. Fig. 4A is the vehicle PBS control group, and Fig. 4B is the plasminogen group. The results show that the expression of LYVE-1 (marked by the arrow) in the tumor tissues of the plasminogen group is significantly less than that of the vehicle PBS control group. It shows that plasminogen can inhibit the formation of lymphatic vessels in colon cancer and metastasis of the cancer tissue.
Figs. 5A-5D show the representative images of H&E staining of tumor mass of the colon cancer model mice after administrating plasminogen by tail vein for 29 days. Figs. 5A and 5C belong to the vehicle PBS control group, and Figs. 5B and 5D belong to the plasminogen group. The results show that the colon cancer subcutaneous tumors in the plasminogen group and the vehicle PBS control group have different degrees of necrosis, and the plasminogen group has more serious necrosis and wider necrosis area than the vehicle PBS control group. In addition, the number of tumor cells in the non-necrotic area of the plasminogen group is significantly less than that of the vehicle PBS control group.

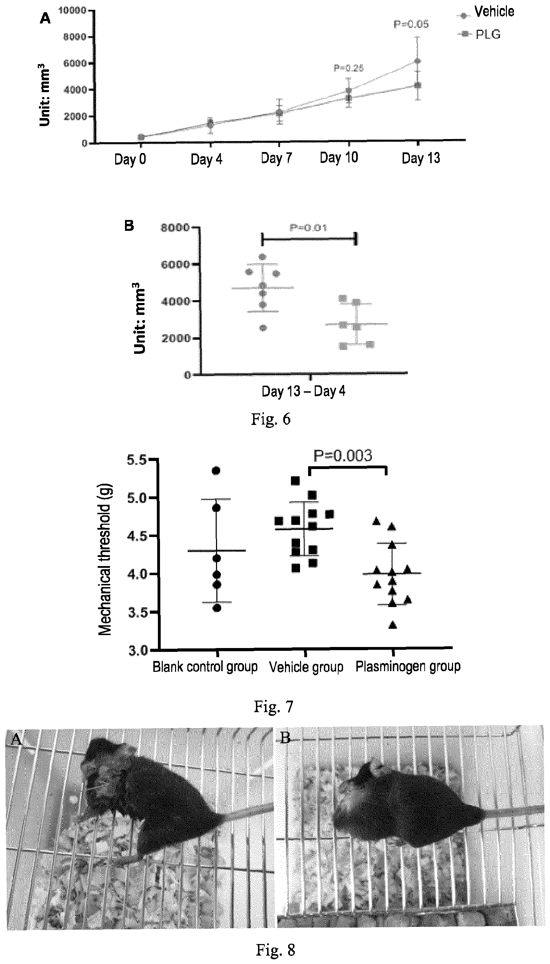

Figs. 6A-6B show the measurement results of tumor volumes of the colon cancer model mice after administrating plasminogen or vehicle, and the results of tumor volume differences between the two groups of mice on Day 13 and Day 4. Fig. 6A shows the measurement results of tumor volumes, and Fig. 6B shows the difference results of tumor volumes between the two groups of mice on Day 13 and Day 4. The results show that the tumor volume of mice in the vehicle group is larger than that in the plasminogen group on Day 7, Day 10 and Day 13; and the statistical difference is close to significant on Day 13 (P=0.05). The differences in tumor volume on Day 13 and Day 4 between the vehicle group and the plasminogen group are compared and analyzed. The results show that the increased tumor volume of mice in the vehicle group is significantly greater than that of mice in the plasminogen group, and the difference is statistically significant (P=0.01). The above results indicate that plasminogen can inhibit tumor growth in colon cancer model mice.
Fig. 7 shows the test results of mechanical touch evoked pain sensing ability of the lung cancer model mice on Day 7 after administrating plasminogen by tail vein. The results show that the pain threshold of mice in the blank control group is normal, and the pain threshold of mice in the vehicle group is increased; the pain threshold of mice in the plasminogen group is significantly lower than that of mice in the vehicle group, and the statistical P value of the two groups is 0.003, and the pain threshold of mice in the plasminogen group is close to that of mice in the blank control group. The results show that plasminogen can significantly improve the pain sensation of the lung cancer model mice.
Figs. 8A-B show the representative images of the lung cancer model mice on Day 20 after administrating plasminogen by tail vein. Fig. 8A is the vehicle control group, and Fig. 8B is the plasminogen group. As shown by the images, on Day 20 the mice in the vehicle PBS control group erected their hairs, their movements were sluggish, their spirits were listless, their tumors were severely ruptured, and the tumor wounds were obviously festered and bleeding (marked by arrows); while the mice in the plasminogen group were able to move freely and in a good state of mind, the tumor had no obvious ulceration phenomenon, and there was a small amount of scab on the epidermis of the tumor. The above results indicate that plasminogen can improve the general physical condition and mental state of the lung cancer model mice.

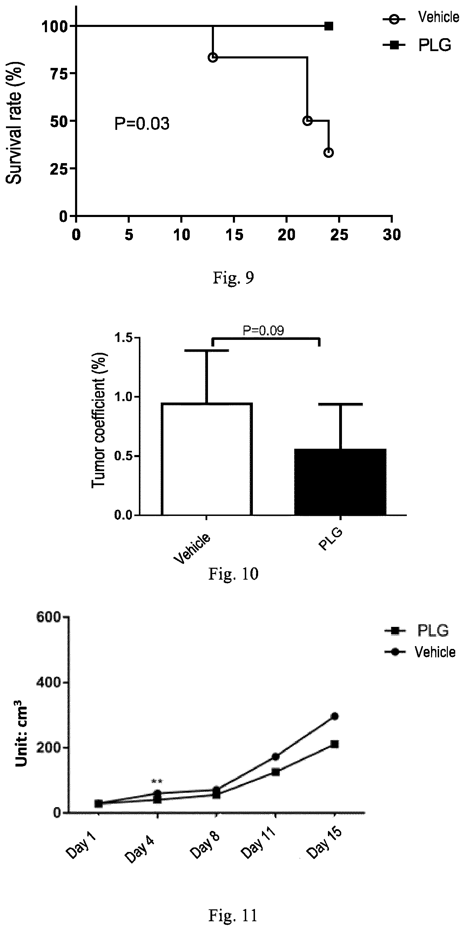

Fig. 9 shows the survival curve of the lung cancer model mice after administrating plasminogen by tail vein for 24 days. It can be seen from the survival curve that, the survival rate of mice in the vehicle control group during the observation period is significantly lower than that of mice in the plasminogen group, and the statistical difference is significant (P=0.03). The results show that plasminogen can improve the survival rate of the lung cancer model mice and prolong the survival time of the mice.
Fig. 10 shows the tumor coefficient results of the lung cancer model mice after administrating plasminogen or vehicle for 14 days. The results show that the tumor coefficient of mice in the plasminogen group is significantly smaller than that of mice in the vehicle group, and the statistical difference is close to significant (P=0.09).
Fig. 11 shows the results of tumor volumes of the lung cancer model mice after administrating plasminogen or vehicle for 14 days. The results show that the tumor volume of mice in the plasminogen group is significantly smaller than that of mice in the vehicle group, and the statistical difference is extremely significant on Day 4 (** indicates P<0.01).

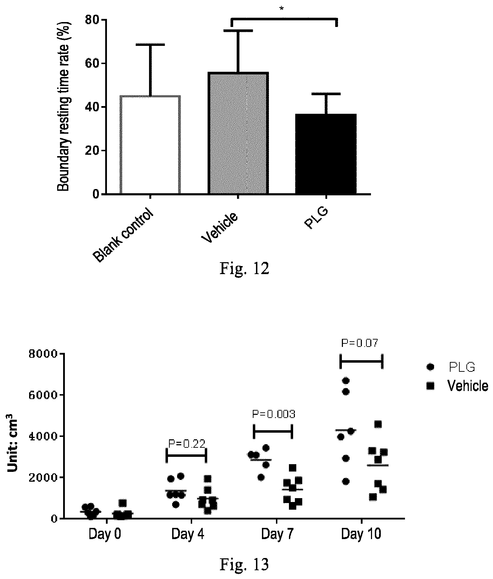

Fig. 12 shows the results of the boundary resting time rate in the open-field test for the lung cancer model mice after administrating plasminogen or vehicle for 7 days. The results show that the blank control mice have a certain boundary resting time rate; the boundary resting time rate of mice in the vehicle group is significantly higher than that of mice in the blank control group; the boundary resting time rate of mice in the plasminogen group is significantly less than that of mice in the vehicle group, and statistical difference is significant (* means P<0.05). The results indicate that plasminogen can promote the recovery of activity and behavior in the lung cancer model mice.
Fig. 13 shows the measurement results of tumor volumes of the lung cancer model mice after administrating plasminogen or vehicle. The results show that the tumor volume of mice in the plasminogen group is significantly smaller than that of mice in the vehicle group at each measurement time point, the statistical P value is 0.22 on Day 4 after administration, the statistical P value is 0.003 on Day 7 after administration, and the P value is 0.07 on Day 10 after administration. The above results show that plasminogen can significantly inhibit tumor growth in the lung cancer model mice.

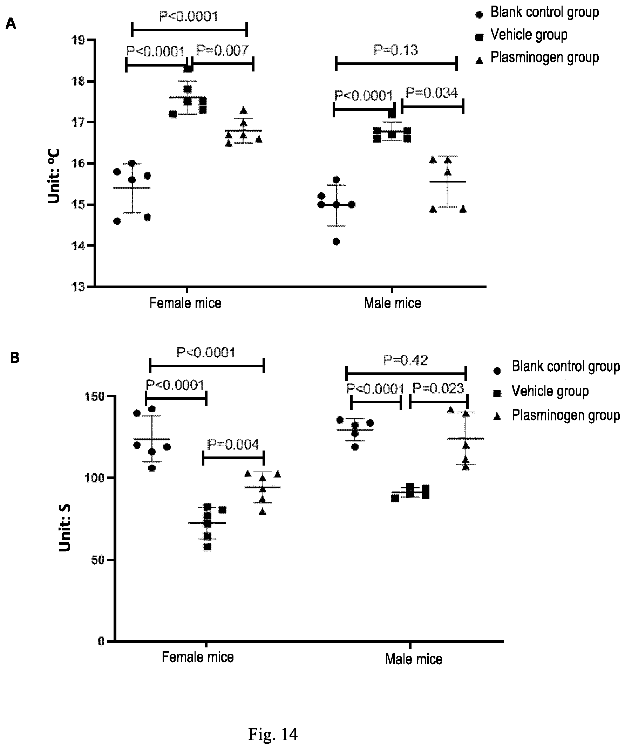

Figs. 14A-14B show the detection results of hot-cold plate analgesia meter for the cancer pain model mice. Fig. 14A shows the statistical results of the temperature when the rear foot of the mouse was contracted to lift during the test, and Fig. 14B shows the statistical results of the time when the rear foot of the mouse was contracted to lift during the test. The results show that the temperature when the rear foot of the mouse is contracted to lift in the vehicle group is significantly higher than that in the blank control group, and the temperature when the rear foot of the mouse is contracted to lift in the plasminogen group is significantly lower than that in the vehicle group, and is close to that in the blank control group. The results of statistical analysis show a comparison between the female mice in the plasminogen group and the vehicle group, P=0.007; and a comparison between the male mice in the plasminogen group and the vehicle group, P=0.043. The time when the rear foot of the mouse is contracted to lift in the vehicle group is significantly earlier than that in the blank control group, and the time when the rear foot of the mouse is contracted to lift in the plasminogen group is significantly later than that in the vehicle group, and is close to that in the blank control group. The results of statistical analysis show a comparison between the female mice in the plasminogen group and the vehicle group, P=0.004; and a comparison between the male mice in the plasminogen group and the vehicle group, P=0.023. The above results show that plasminogen can relieve the pain sensation of the cancer pain model mice.

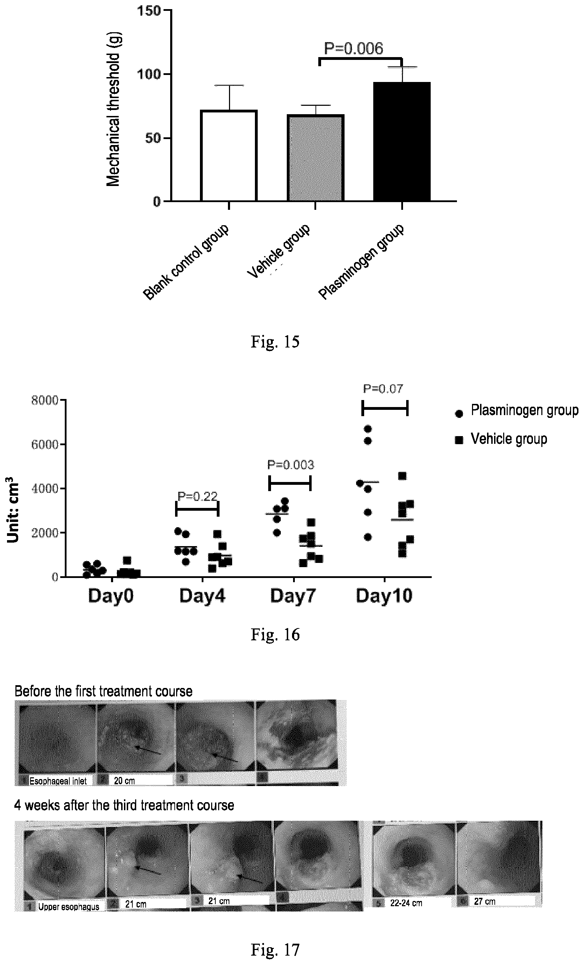

Fig. 15 shows the measurement results of the pincher analgesia meter for the cancer pain model mice after administrating plasminogen. The results show that the pain threshold of mice in the plasminogen group is significantly higher than that of mice in the vehicle group, and the P value of statistical analysis is 0.006. The results show that plasminogen can relieve the pain sensation of the cancer pain model mice.
Fig. 16 shows the measurement results of tumor volumes of the lung cancer model mice after administrating plasminogen. The results show that the tumor volume of mice in the plasminogen group is not different from that of mice in the vehicle group on Day 0, while the tumor volume of mice in the plasminogen group is significantly smaller than that of mice in the vehicle group respectively on Day 4, Day 7 and Day 10; and the P values of statistical analysis are 0.22, 0.003, and 0.07, respectively. The results show that plasminogen can obviously inhibit the growth of lung cancer tumor.
Fig. 17 shows the esophageal electronic gastroscopy examination results after administrating plasminogen and chemotherapy to a patient with esophageal cancer. Before the first course of treatment and 4 weeks after the end of the third course of treatment, the electronic gastroscopy was performed on the patient. The results show that before administration, an esophageal tumor mass (marked by the arrow) can be seen in the esophagus at a position 20 cm-30 cm away from the incisors of the patient, involving the esophagus for 3/4 of the circumference, and the tumor mass is fragile and easy to bleed; the esophagus is narrow, and the gastroscopy body barely passes through it. Four weeks after the third course of treatment, the results of electronic gastroscopy show that a 0.8 cm-size raised lesion (marked by the arrow) can be seen in the esophagus at a position 21 cm away from the incisors, the mucosa is smooth; and a 2 cm-size ulcerative raised lesion can be seen in the esophagus at a position 22-24 cm away from the incisors, involving the esophagus for 2/5 of the circumference; a 0.3 cm-size polyp can be seen at a position 27 cm away from the incisors, the mucosa is smooth; and the three lesions are not connected to each other. The volume of the esophageal cancer mass of the patient is significantly reduced after treatment with plasminogen and chemotherapy.

### DETAILED DESCRIPTION OF THE APPLICATION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and vascular endothelial cells, and may directly activate plasminogen without the need for fibrin as a cofactor. Plasminogen (PLG) is synthesized by the liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and II, etc.; converting plasminogen into plasmin; hydrolyzing complements, etc. Fibrinolysis inhibitors: including a plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by the liver, and binds to PL in a ratio of 1:1 to form a complex to inhibit the activity of PL; FXIII makes α2-AP covalently bound to fibrin, reducing the sensitivity of fibrin to PL. Substances that inhibit the activity of the fibrinolytic system in vivo include: PAI-1, a complement C1 inhibitor; α2 antiplasmin; and α2 macroglobulin.

The term "component of plasminogen activation pathway" of the present application encompasses:
1. plasminogen, Lys-plasminogen, Glu-plasminogen, micro-plasminogen, delta-plasminogen; a variants or analogs thereof;
2. plasmin and a variant or analog thereof; and
3. plasminogen activators, such as tPA and uPA, and tPA or uPA variants and analogs comprising one or more domains of tPA or uPA, such as one or more kringle domains and serine protease domains.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the present application encompasses a protein having at least 75%, 80%, 85%, 90%, 95%, 96%, 97%, 98% or 99% sequence identity with SEQ ID NO: 2, 6, 8, 10 or 12, and still retaining plasminogen activity. For example, a "plasminogen variant" of the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining plasminogen activity. Particularly, the plasminogen variants of the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid.

The plasminogen of the present application may be a human plasminogen ortholog from a primate or rodent, or a variant thereof still retaining plasminogen activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and serine protease domains). For example, "variants" and "analogs" of plasminogen encompass "variants" and "analogs" of plasminogen comprising one or more plasminogen domains (e.g., one or more kringle domains and serine protease domains), such as mini-plasminogen. "Variants" and "analogs" of plasmin encompass "variants" and "analogs" of plasmin comprising one or more plasmin domains (e.g., one or more kringle domains and serine protease domains), such as mini-plasmin, and delta-plasmin.

Whether the "variant" or "analog" of the above plasminogen, plasmin, tPA or uPA respectively has the activity of plasminogen, plasmin, tPA or uPA, or whether the "variant" or "analog" provides substantially similar effect to plasminogen, plasmin, tPA or uPA, may be detected by methods known in the art, for example, it is measured by the level of activated plasmin activity based on enzymography, ELISA (enzyme-linked immunosorbent assay), and FACS (fluorescence-activated cell sorting method), for example, it may be detected by referring to a method selected from the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5):1625-33; Gravanis I, Tsirka SE (February 2008). "Tissue-type plasminogen activator as a therapeutic target in stroke". Expert Opinion on Therapeutic Targets. 12(2):159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" of the present application is a plasminogen. In some embodiments, the plasminogen is selected from the group consisting of: Glu-plasminogen, Lys-plasminogen, mini-plasminogen, micro-plasminogen, delta-plasminogen. In some embodiments, the plasminogen is a plasminogen protein comprising one or more kringle domains. In some embodiments, the plasminogen is a plasminogen protein comprising a serine protease domain. In some embodiments, the plasminogen is a plasminogen protein comprising a serine protease domain represented by SEQ ID NO: 14. In some embodiments, the plasminogen is a plasminogen protein comprising a serine protease domain having at least 80%, 85%, 90%, 95%, 96%, 97%, 98%, 99% identity with the amino acid sequence represented by SEQ ID NO: 14. In some embodiments, the plasminogen is natural or synthetic human full-length plasminogen. In some embodiments, the amino acid sequence of the plasminogen is represented by SEQ ID NO: 2, 6, 8, 10 or 12. In some embodiments, the plasminogen is a human natural plasminogen. In some embodiments, the plasminogen is a human natural plasminogen represented by SEQ ID NO: 2.

In some embodiments, the plasminogen is a protein having at least 75%, 80%, 85%, 90%, 95%, 96%, 97%, 98% or 99% sequence identity with SEQ ID NO: 2, 6, 8, 10 or 12, and still having plasminogen activity. In some embodiments, the plasminogen is a conservatively substituted variant of SEQ ID NO: 2, 6, 8, 10 or 12.

"Plasminogen activity" of the present application refers to the lysine binding activity and proteolytic activity of plasminogen to its receptor or substrate.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans^{[1]}. In addition, plasmin may activate some metalloproteinase precursors (pro-MMPs) to form active metalloproteinases (MMPs). Therefore, plasmin is considered to be an important upstream regulator of extracellular proteolysis^{[2-3]}. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity^{[4-5]}.

Plasminogen is a single-chain glycoprotein consisting of 791 amino acids with a molecular weight of approximately 92 kDa^{[6-7]}. Plasminogen is mainly synthesized in the liver, and is abundantly present in the extracellular fluid. The content of plasminogen in plasma is approximately 2 µM. Plasminogen is thus a huge potential source of proteolytic activity in tissues and body fluids^{[8-9]}. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of two-chain protease plasmin linked by disulfide^{[10]}. The amino-terminal part of plasminogen comprises five homologous tri-cycles, i.e., so-called kringles domains (kringle 1, kringle 2, kringle 3, kringle 4, kringle 5), and the carboxy-terminal part comprises the protease domain. Some kringles comprise lysine binding sites that mediate the specific interaction of plasminogen with fibrin and its inhibitor α2-AP, and are therefore also called lysine binding domain. In the present application, the lysine binding domain refers to the structural region consisting of any one, two, three, four or five kringles selected from kringle 1, kringle 2, kringle 3, kringle 4 and kringle 5. Multiple recent discoveries of a 38kDa fragment of plasminogen, including kringles1-4, are potent inhibitors of angiogenesis. A recently found plasminogen is a 38 kDa fragment, including kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis^{[11]}. Plasmin also has substrate specificity for several components of the ECM, including laminin, fibronectin, proteoglycans, and gelatin, indicating that plasmin also plays an important role in ECM remodeling^{[7,12-13]}. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis^{[14]}. In addition, plasmin has the ability to activate certain latent forms of growth factors^{[15-17]}. In vitro, plasmin also hydrolyzes components of the complement system, and releases chemotactic complement fragments.

"Plasmin" is a very important enzyme present in the blood that hydrolyzes fibrin clots into fibrin degradation products and D-dimers.

"Plasminogen" is the zymogen form of plasmin. According to the sequence in swiss prot, it consists of 810 amino acids calculating by the natural human plasminogen amino acid sequence (SEQ ID NO: 4) containing the signal peptide, and the molecular weight is about 90 kD, and it is a glycoprotein mainly synthesized in the liver and capable of circulating in the blood, the cDNA sequence encoding this amino acid sequence is represented by SEQ ID NO: 3. Full-length plasminogen contains seven domains: a C-terminal serine protease domain (or protease domain for short), an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle 1 comprises residues Cys103-Cys181, Kringle 2 comprises residues Glu184-Cys262, Kringle 3 comprises residues Cys275-Cys352, Kringle 4 comprises residues Cys377-Cys454, and Kringle 5 comprises residues Cys481-Cys560. According to NCBI data, the serine protease domain comprises residues Val581-Arg804.

Glu-plasminogen is human natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. In vivo, there is also a Lys-plasminogen produced by the hydrolysis of the peptide bond between amino acids 76 and 77 of Glu-plasminogen, the amino acid sequence is represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and serine protease domains^{[18-19]}. The amino acid sequence of delta-plasminogen (SEQ ID NO: 8) is reported in a literature^{[19]}, and the cDNA sequence encoding this amino acid sequence is represented by SEQ ID NO: 7. Mini-plasminogen consists of Kringle5 and serine protease domains, and it is reported that it comprises residues Val443-Asn791 (with the Glu residue of the Glu-plasminogen sequence without the signal peptide as the starting amino acid) ^{[20]}, its amino acid sequence is represented by SEQ ID NO: 10, and the cDNA sequence encoding this amino acid sequence is represented by SEQ ID NO: 9. Micro-plasminogen comprises only a serine protease domain, and it is reported that its amino acid sequence comprises residues Ala543-Asn791 (with the Glu residue of the Glu-plasminogen sequence without the signal peptide as the starting amino acid) ^{[21]}; additionally, it is disclosed in patent document CN102154253A that its sequence comprises residues Lys531-Asn791 (with the Glu residue of the Glu-plasminogen sequence without the signal peptide as the starting amino acid); in the present patent application, the sequence refers to the patent document CN102154253A, the amino acid sequence is represented by SEQ ID NO: 12, and the cDNA sequence encoding this amino acid sequence is represented by SEQ ID NO: 11. The amino acid sequence of the serine protease domain in this application is represented by SEQ ID NO: 14, and the cDNA sequence encoding the amino acid sequence is represented by SEQ ID NO: 13.

During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the Kringle domain may bind to a lysine residue present on a receptor and substrate. The plasminogen of the present application encompasses a plasminogen comprising one or more Kringle domains selected from Kringle 1, Kringle 2, Kringle 3, Kringle 4 and Kringle 5.

The "serine protease domain", also known as the protease domain, is a domain by which plasminogen performs proteolytic function. The technical solutions related to plasminogen of the present application encompass all technical solutions of plasminogen comprising a serine protease domain. The plasminogen fragment comprising serine protease domain of the present application is a protein comprising the serine protease domain of plasminogen. In some embodiments, the plasminogen fragment comprising a serine protease domain described herein is a protein comprising the amino acid sequence represented by SEQ ID NO: 14. In some embodiments, the plasminogen fragment comprising a serine protease domain of the present application is a protein comprising the amino acid sequence having at least 80%, 90%, 95%, 96%, 97%, 98%, 99% identity with SEQ ID NO: 14. In some embodiments, the plasminogen comprises a conservatively substituted variant of SEQ ID NO: 14.

At present, the methods for measuring plasminogen and its activity (e.g., proteolytic activity) comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the plasminogen in the plasma to be detected is converted into plasmin under the action of SK, and PLM acts on the chromogenic substrate; subsequently, the detection by spectrophotometer indicates that the increase in absorbance is proportional to plasminogen activity. In addition, the plasminogen activity (e.g., proteolytic activity) may also be detected by immunochemical method, gel electrophoresis, immunoturbidimetry, and radioimmunoassay.

Angiostatin has a molecular weight of 38 kD, and is a part of plasminogen^{[22-23]}. Its amino acid sequence shares 98% homology with Kringle1-Kringle4 of the five Kringles of plasminogen. Angiostatin is formed in vivo by cleavage of plasminogen by elastase, different matrix metalloproteinases and other proteolytic enzymes^{[24-25]}. Angiostatin can bind to various proteins including angiomotin, annexin II, tPA and CD26^{[26]}, and studies have shown that angiostatin can interact with these proteins to inhibit angiogenesis.

Studies have found that angiostatin can specifically inhibit proliferation, migration of vascular endothelial cells and induce apoptosis, and the mechanism may be closely related to the lysine-binding activity of kringle in angiostatin. Plasminogen Kringle5 (K5) can also significantly inhibit the growth of endothelial cells, and its activity is significantly higher than that of angiostatin. However, studies have shown that although K5 also has lysine-binding activity, its inhibitory effect on angiogenesis does not depend on lysine-binding activity, and the K5 structure has a unique mechanism of action ^{[27]}. As a large number of follow-up studies have shown that the Kringle structures of different forms of plasminogen have exhibited different inhibitory activities on angiogenesis, the concept of angiostatin has been expanded from a single protein to a class of proteins (angiostatin isoforms or angiostatin related proteins), including a series of fragments containing different Kingle structures and having similar biological activity of plasminogen ^{[28-29]}.

In 1971, Professor Judah Folkman of Harvard University proposed for the first time the theory of cutting off tumor blood vessels and starving cancer cells to death ^{[30]}; and in 1994 angiostatin was discovered as a molecule with the effect of inhibiting angiogenesis, which is produced through enzymatic hydrolysis of endogenous plasminogen by MMPs ^{[21]}. Later, multiple research data showed that angiostatin can inhibit tumor growth and metastasis by inhibiting angiogenesis. However, in the research of Professor Folkman et al., it is clearly pointed out that only angiostatin or kringle5 has these effects, and the complete plasminogen does not have the effect of inhibiting angiogenesis and inhibiting tumors ^{[23,31,32]}. Therefore, Professor Folkman et al. proposed that angiostatin can be used clinically to inhibit tumor angiogenesis, thereby inhibiting cancer. However, after about 20 years of hard work, angiostatin and its analogues have not become a class of effective drugs for inhibiting tumor angiogenesis as people imagined ^{[33-34]}. On the contrary, inhibitors such as VEGF antibodies have gradually become a mainstream drug development strategy for inhibiting tumor angiogenesis since around year 2000, and have been widely used clinically such as colon cancer ^{[35]}. It can be seen from the above that, the idea of inhibiting angiogenesis to inhibit tumors is correct, but the method of directly using angiostain and its analogs to inhibit tumors may have defects.

However, in our study, we clearly observed that intact plasminogen has the effect of inhibiting angiogenesis, tumor growth and metastasis. Especially when we basically repeated the experiments published by Professor Folkman et al. in "Cell" in 1994 and "Nature Medicine" in 1996 ^{[23,36]}, only after increasing the injection amount of plasminogen, we clearly obtained different results from Professor Folkman et al.; particularly, after injecting human plasminogen, it can not only significantly reduce angiogenesis, but also inhibit the increase of intestinal cancer tumor volume, and can significantly prolong the survival period and survival rate of mice under the condition of LLC tumors. It is worth pointing out that, we increased the injection amount of plasminogen in these experiments, because we believe that in the "Cell" and other articles, it is questionable that the same mass of plasminogen was used in the control for angiostatin - as angiostatin is the product of plasminogen proteolysis, at least the same molar amount of plasminogen instead of the same mass of plasminogen should be used in the control for angiostatin. Anyway, these results suggest that direct administration of plasminogen itself inhibits angiogenesis. This effect is likely to be that the extra plasminogen in the body is degraded by proteolytic enzymes such as MMPs and elastase accumulated in the tumor microenvironment, thereby promoting the production of high amounts of angiostatin and/or its analogs, and then exhibiting the function of inhibiting tumor angiogenesis. That is to say, the inhibition of angiogenesis can be achieved not by directly increasing the angiostatin and/or its analogues in the body, but directly by regulating the plasminogen in the body. This opens up a whole new therapeutic strategy for tumor suppression.

The plasminogen of the present application encompasses fragments of plasminogen having plasminogen activity, e.g., fragments of plasminogen comprising one or more distinct Kingle domains, or fragments of plasminogen comprising variants with similar biological activity to one or more distinct Kingle domains.

"Ortholog or orthologs" refer to homologs between different species, including both protein homologs and DNA homologs, also known as orthologs and vertical homologs; particularly it refers to proteins or genes evolved from the same ancestral gene in different species. The plasminogen of the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having not less than 60%, preferably not less than 75%, more preferably not less than 85%, or even most preferably not less than 90% amino acid identity determined by BLAST or FASTA algorithm, and having the same or substantially similar properties or functions as the natural or parent protein or enzyme.

In the present application, "plasmin" and "fibrinolytic enzyme" can be used interchangeably, and have the same meaning; "plasminogen" and "fibrinolytic zymogen" can be used interchangeably with the same meaning. Those skilled in the art may understand that, all technical solutions of plasminogen of the present application are applicable to plasmin, thus the technical solutions described in the present application encompass plasminogen and plasmin.

"A compound capable of directly activating plasminogen, or indirectly activating plasminogen by activating a upstream component of plasminogen activation pathway", refers to any compound capable of directly activating plasminogen, or indirectly activating plasminogen by activating a upstream component of plasminogen activation pathway, such as tPA, uPA, streptokinase, saruplase, alteplase, reteplase, tenecteplase, anistreplase, monteplase, lanoteplase, pamiteplase, and staphylokinase.

The "antagonist of a fibrinolysis inhibitor" of the present application is a compound that antagonizes, weakens, blocks, or prevents the action of a fibrinolysis inhibitor. Such fibrinolysis inhibitors are e.g., PAI-1, complement C1 inhibitor, α2 antiplasmin, and α2 macroglobulin. Such an antagonist is: e.g., an antibody of PAI-1, complement C1 inhibitor, α2 antiplasmin, or α2 macroglobulin; or an antisense RNA or small RNA blocking or downregulating the expression of such as PAI-1, complement C1 inhibitor, α2 antiplasmin or α2 macroglobulin; or a compound occupying the binding site of PAI-1, complement C1 inhibitor, α2 antiplasmin, or α2 macroglobulin but without the function of PAI-1, complement C1 inhibitor, α2 antiplasmin, or α2 macroglobulin; or a compound blocking the binding and/or active domains of PAI-1, complement C1 inhibitor, α2 antiplasmin, or α2 macroglobulin.

In this application, the "deficiency" of plasminogen means that the content or activity of plasminogen in the subject is lower than that of a normal person, and is low enough to affect the normal physiological functions of the subject; the "lack" of plasminogen means that the content or activity of plasminogen in the subject is significantly lower than that of a normal person, even the activity or expression is minimal, and normal physiological function can only be maintained by external source of plasminogen.

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or non-reducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:
Fraction X/Y times 100;
wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

As used herein, the terms "treatment" and "treating" refer to obtaining a desired pharmacological and/or physiological effect. The effect may be complete or partial prevention of the disease or symptoms thereof, and/or partial or complete cure of the disease and/or symptoms thereof; and includes: (a) preventing the occurrence of the disease in a subject, who may have predisposition of the disease, but is not yet diagnosed as having the disease; (b) inhibiting the disease, i.e., blocking its development; and (c) alleviating the disease and/or symptoms thereof, i.e., causing regression of the disease and/or symptoms thereof.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of plasminogen sufficient to prevent and/or treat a disease when administrated to a mammal or other subject for treating the disease. A "therapeutically effective amount" will vary depending on the plasminogen used, the severity of the disease and/or symptoms thereof in the subject to be treated, as well as the age, weight, and the like.

### Preparation of the Plasminogen of the Present Application

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen of the present application may be produced by standard recombinant methods. For example, a nucleic acid encoding plasminogen is inserted into an expression vector to operably link to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector typically replicates in a host organism as an episome or as an incorporated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a subject protein-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., *S*. *cerevisiae*) and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization

In addition to microorganisms, mammalian cells (e.g., mammalian cells grown in in vitro cell culture) may also be used to express and produce the compound of the application (e.g., plasminogen), See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the compound of the application (e.g., plasminogen), and the like.

### Medicament Formulation

A therapeutic formulation may be prepared by mixing the plasminogen having desired purity with an optional pharmaceutical carrier, excipient, or stabilizer (Remington's Pharmaceutical Sciences, 16th edition, Osol, A. ed. (1980)), to form a lyophilized formulation or aqueous solution. An acceptable carrier, excipient, or stabilizer is non-toxic to a recipient at the employed dosage and concentration, including buffers such as phosphate, citrate and other organic acids; antioxidants such as ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG).

The formulations of the present application may also contain not less than one active compound as required for the particular condition to be treated, preferably those compounds that are complementary in activity and do not have side effects with each other. For example, antineoplastic medicaments, liver protection medicaments, hormone medicaments, etc.

The plasminogen of the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The plasminogen of the present application for in vivo administration must be sterile. This may be easily achieved by filtration through sterilizing filters before or after lyophilization and reformulation.

The plasminogen of the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot^{™} (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for not less than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix composition.

### Administration and Dosage

Administration of the pharmaceutical composition of the present application may be accomplished by different means, e.g., intravenously, intraperitoneally, subcutaneously, intracranially, intrathecally, intraarterally (e.g., via the carotid artery), or intramuscularly.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other concomitantly administrated medicaments. The dosage range of the pharmaceutical composition comprising the plasminogen of the present application may be, for example, about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 1-10 mg/kg on consecutive days. Real-time evaluation of therapeutic efficacy and safety is required during the administration of the medicament of the present application.

### Article of Manufacture or Kit

One embodiment of the present application relates to an article of manufacture or kit comprising the plasminogen or plasmin of the present application for treating a tumor. The article of manufacture preferably comprises a container, and a label or package insert. Suitable containers are bottles, vials, syringes, etc. The container may be made of various materials such as glass or plastic. The container contains a composition which is effective for treatment of the disease or condition of the present application and has a sterile access port (e.g., the container may be an intravenous solution pack or vial containing a stopper penetrable by a hypodermic needle). At least one active agent in the composition is plasminogen/plasmin. The label on or attached to the container indicates that the composition is used for treatment of tumors of the present application. The article of manufacture may further comprise a second container containing a pharmaceutically acceptable buffer, such as phosphate buffered saline, Ringer's solution, and dextrose solution. It may further contain other materials required from a commercial and user standpoint, including other buffers, diluents, filters, needles and syringes. In addition, the article of manufacture comprises a package insert with instructions for use, including, for example, instructing the user of the composition to administrate the plasminogen composition to the patient along with other medicaments for treatment of concomitant diseases.

### EXAMPLE

The plasminogen used in the following examples is human plasminogen derived from plasma of a human donor, and it is purified from human plasma by a method in which part of the process was optimized based on methods described in reference literatures ^{[37-39]}, wherein human Lys-plasminogen and Glu-plasminogen are >98%.

### Example 1: Plasminogen Inhibits the Growth of Colon Cancer

CT26. WT mouse colon cancer cells belong to an undifferentiated colon cancer cell line induced by N-nitroso-N-methylurethane-(NNMU). The cell line formed by its clone was named CT26. WT (ATCC CRL-2638).

CT26. WT (ATCC CRL-2638) mouse colon cancer cells were purchased from Shanghai Institutes for Biological Sciences, Chinese Academy of Sciences (hereinafter the cells are referred to as CT26). CT26 cells were placed in PRIM-1640 (GIBCO, Cat. No. 31800022) culture medium containing 10% fetal bovine serum, then culturing in 5% CO₂ incubator at 37°C. After the growth of the cells in the culture flask reached 90% confluency, the cells were digested with trypsin (0.02% EDTA added to 0.05% trypsin) (Sigma, 74799), then washing 3 times with normal saline, and resuspending in normal saline at a concentration of 10⁷ cells/ml.

14 male Balb/c mice of 7-9 week-age were anesthetized by intraperitoneal injection of pentobarbital sodium at 50 mg/kg body weight. After anesthesia, the mice were subcutaneously inoculated with 10⁶ cells/100 µl CT26 resuspension solution on the back of the mouse with a 27-gauge needle, then measuring the volume of the subcutaneous tumor (length × width² × 0.52) ^{[40-41]} of the mice every day with a vernier caliper for 7 consecutive days. According to the tumor volumes on Day 7, the mice were randomly divided into two groups, the plasminogen group and the PBS control group, with 7 mice in each group. The mice were administrated from Day 8 after CT26 inoculation, and it was designated as Day 1 of administration; particularly, human plasminogen was injected into the mice of the plasminogen group by tail vein at 1 mg/0.1mL/mouse/day, and the same volume of PBS (20 mM citric acid-sodium citrate, 2% arginine hydrochloride, 3% mannitol, pH 7.4, the same as below) was injected into the mice of the vehicle PBS group by tail vein, administrating for 23 consecutive days. On Days 1, 4, 7, 10, 13, 17, 20 and 24, the length and width of the tumor were measured, and the tumor volume was calculated.

### Tumor Volume

The results show that, the colonic tumor growth rate of mice in the plasminogen group is significantly smaller than that of mice in the vehicle PBS control group during the administration period, and the statistical difference in the tumor size of the two groups of mice is close to significant on Day 20 and Day 24 (P=0.06); The tumor volume of mice in the plasminogen group is reduced by 40% on Day 17, 26% on Day 20, and 32% on Day 24 as compared with that of mice in the vehicle PBS control group (Fig. 1).

### Tumor Volume Difference

The difference between the tumor volume on Day 20 and the tumor volumes on Day 10 and Day 13, and the difference between the tumor volume on Day 24 and the tumor volume on Day 10 were calculated. The results show that, the tumor volume increase of mice in the plasminogen group is significantly smaller than that of mice in the vehicle PBS control group, and the difference is statistically significant (* indicates P<0.05) (Fig. 2).

The above results show that plasminogen can inhibit the growth of colon cancer cells.

### Example 2: Plasminogen Inhibits the Formation of Microvessels in Colon Cancer Tissue

14 male Balb/c mice of 7-9 week-age were anesthetized by intraperitoneal injection of pentobarbital sodium at 50 mg/kg body weight. After anesthesia, the mice were subcutaneously inoculated with 10⁶ cells/100 µl CT26 resuspension solution on the back of the mouse with a 27-gauge needle, then measuring the volume of the subcutaneous tumor (length × width² × 0.52) ^{[40-41]} of the mice every day with a vernier caliper for 7 consecutive days. According to the tumor volumes on Day 7, the mice in the model group were randomly divided into two groups, the plasminogen group and the PBS control group, with 7 mice in each group. The mice were administrated from Day 8 after CT26 inoculation, and it was designated as Day 1 of administration; particularly, human plasminogen was injected into the mice of the plasminogen group by tail vein at 1mg/0.1mL/mouse/day, and the same volume of PBS (20 mM citric acid-sodium citrate, 2% arginine hydrochloride, 3% mannitol, pH 7.4, the same as below) was injected into the mice of the vehicle PBS group by tail vein, administrating for 29 consecutive days. The mice were sacrificed on Day 30, and the tumor tissues were taken to fix in 10% neutral formaldehyde for 24-48 hours, and then dehydrated and embedded. The fixed tissue samples were dehydrated with alcohol gradients and cleared with xylene for embedding in paraffin. The thickness of tumor tissue slices was 4 µm, and the slices were washed once with water after dewaxing and rehydrating; then repairing with citric acid for 30 minutes, cooling at room temperature for 10 minutes, and then rinsing gently with water. The tumor tissue slices were incubated with 3% hydrogen peroxide for 15 minutes, then circling the tissue with a PAP pen, and blocking with 10% goat serum (Vector laboratories, Inc., USA) for 1 hour; after that, the goat serum was discarded. The slices were incubated rabbit-derived anti-CD31 antibody (Abcam, ab28364) overnight at 4°C, then washing twice with PBS, 5 minutes each time; and then incubating with goat anti-rabbit IgG (HRP) antibody (Abcam) secondary antibody at room temperature for 1 hour, washing twice with PBS, 5 minutes each time. The slices were color-developed according to the DAB kit (Vector laboratories, Inc., USA), then washing 3 times with water, counterstaining with hematoxylin for 30 seconds, and returning blue in running water for 5 minutes, and then washing once with PBS. After performing gradient dehydration and making the slices transparent and sealed, the slices were observed under a 200X optical microscope.

CD31 is usually located in vascular endothelial cells, platelets, macrophages and kuffer cells, granulocytes, T/NK cells, lymphocytes, megakaryocytes, osteoclasts, and neutrophils. In immunohistochemistry, CD31 is a marker of vascular endothelial cells, and can be used to assess tumor angiogenesis ^{[42-43]}.

The results show that the expression of CD31 (marked by the arrow) in the tumor mass of mice in the plasminogen group (Fig. 3B) is significantly less than that of mice in the vehicle PBS control group (Fig. 3A). It shows that plasminogen can inhibit the expression of vascular endothelial cell marker CD31 in tumor tissue, suggesting that plasminogen can inhibit the formation of new blood vessels in colon cancer tissues.

### Example 3: Plasminogen Inhibits Lymphangiogenesis in Colon Cancer Tissue

14 male Balb/c mice of 7-9 week-age were anesthetized by intraperitoneal injection of pentobarbital sodium at 50 mg/kg body weight. After anesthesia, the mice were subcutaneously inoculated with 10⁶ cells/100 µl CT26 resuspension solution on the back of the mouse with a 27-gauge needle, then measuring the volume of the subcutaneous tumor (length × width² × 0.52) ^{[40-41]} of the mice every day with a vernier caliper for 7 consecutive days. According to the tumor volumes on Day 7, the mice in the model group were randomly divided into two groups, the plasminogen group and the PBS control group, with 7 mice in each group. The mice were administrated from Day 8 after CT26 inoculation, and it was designated as Day 1 of administration; particularly, human plasminogen was injected into the mice of the plasminogen group by tail vein at 1mg/0.1mL/mouse/day, and the same volume of PBS (20mM citric acid-sodium citrate, 2% arginine hydrochloride, 3% mannitol, pH 7.4, the same as below) was injected into the mice of the vehicle PBS group by tail vein, administrating for 29 consecutive days. The mice were sacrificed on Day 30, and the tumor tissues were taken to fix in 10% neutral formaldehyde for 24-48 hours, and then dehydrated and embedded. The fixed tissue samples were dehydrated with alcohol gradients and cleared with xylene for embedding in paraffin. The thickness of tumor tissue slices was 4 µm, and the slices were washed once with water after dewaxing and rehydrating; then repairing with citric acid for 30 minutes, cooling at room temperature for 10 minutes, and then rinsing gently with water. The tumor tissue slices were incubated with 3% hydrogen peroxide for 15 minutes, then circling the tissue with a PAP pen, and blocking with 10% goat serum (Vector laboratories, Inc., USA) for 1 hour; after that, the goat serum was discarded. The slices were incubated rabbit-derived anti-LYVE-1 antibody (Abeam, ab14917) overnight at 4°C, then washing twice with PBS, 5 minutes each time; and then incubating with goat anti-rabbit IgG (HRP) antibody (Abcam) secondary antibody at room temperature for 1 hour, washing twice with PBS, 5 minutes each time. The slices were color-developed according to the DAB kit (Vector laboratories, Inc., USA), then washing 3 times with water, counterstaining with hematoxylin for 30 seconds, and returning blue in running water for 5 minutes, and then washing once with PBS. After performing gradient dehydration and making the slices transparent and sealed, the slices were observed under a 200X optical microscope.

Lymphatic vessel endothelial hyaluronan receptor 1 (LYVE-1) is a receptor on the surface of lymphatic endothelial cells, and can be used as a marker of lymphatic endothelial cells ^{[44-45]}.

The results show that the expression of LYVE-1 (marked by the arrow) in the tumor mass of mice in the plasminogen group (Fig. 4B) is significantly less than that of mice in the vehicle PBS control group (Fig. 4A). The experimental results show that, plasminogen can inhibit the expression of lymphatic endothelial cell marker LYVE-1 in the tumor, suggesting that plasminogen can inhibit the formation of lymphatic vessels in colon cancer tissues.

### Example 4: Plasminogen Promotes the Necrosis of Colon Cancer Cells

14 male Balb/c mice of 7-9 week-age were anesthetized by intraperitoneal injection of pentobarbital sodium at 50 mg/kg body weight. After anesthesia, the mice were subcutaneously inoculated with 10⁶ cells/100 µl CT26 resuspension solution on the back of the mouse with a 27-gauge needle, then measuring the volume of the subcutaneous tumor (length × width² × 0.52) ^{[40-41]} of the mice every day with a vernier caliper for 7 consecutive days. According to the tumor volumes on Day 7, the mice in the model group were randomly divided into two groups, the plasminogen group and the PBS control group, with 7 mice in each group. The mice were administrated from Day 8 after CT26 inoculation, and it was designated as Day 1 of administration; particularly, human plasminogen was injected into the mice of the plasminogen group by tail vein at 1 mg/0.1mL/mouse/day, and the same volume of PBS (20 mM citric acid-sodium citrate, 2% arginine hydrochloride, 3% mannitol, pH 7.4, the same as below) was injected into the mice of the vehicle PBS group by tail vein, administrating for 29 consecutive days. The mice were sacrificed on Day 30, and the tumor tissues were taken to fix in 10% neutral formaldehyde for 24-48 hours. The fixed tumor tissues were dehydrated with alcohol gradients and cleared with xylene for embedding in paraffin. The thickness of tissue slices was 3 µm, and the slices were dewaxed and rehydrated, then staining with hematoxylin and eosin (H&E staining), differentiating with 1% hydrochloric acid alcohol, returning blue with ammonia water. After performing gradient dehydration and making the slices sealed, the slices were observed under a 200X optical microscope. Fig. 5C and Fig. 5D are enlarged pictures of the framed areas in Fig. 5A and Fig. 5B respectively.

The results show that, the subcutaneous tumor mass of colon cancer of mice in the plasminogen group (Fig. 5B) and the vehicle PBS control group (Fig. 5A) appear different degrees of necrosis (arrows), and the necrosis in the plasminogen group is more serious with larger area of necrosis as compared with the vehicle PBS group. In addition, the number of tumor cells in the non-necrotic area of mice in the plasminogen group (Fig. 5D) is significantly less than that of mice in the vehicle PBS control group (Fig. 5C). It shows that plasminogen can promote the necrosis of colon cancer cells.

### Example 5: Plasminogen Inhibits Tumor Growth in Colon Cancer Model Mice

21 female Balb/c mice of 9-week age were weighed and randomly divided into two groups, i.e., 9 mice in the blank control group, and 18 mice in the model group. After grouping, all mice in the model group were anesthetized with 2% isoflurane, disinfecting the axilla of one side and subcutaneously injecting 1 ×10⁶ cells of CT-26 cell suspension solution, then observing for 7 days. On Day 8 after tumor cell inoculation, the tumor volumes were measured for all mice. After the tumor volume of the mice reached more than 100 mm³, all mice were weighed, and randomly grouped according to the results of body weight and tumor volume, i.e., 9 mice in the vehicle group, and 9 mice in the plasminogen group. After the grouping was completed, human plasminogen was injected into mice of the plasminogen group by tail vein at 1 mg/0.1mL/mouse/day, and the same volume of vehicle PBS was injected into the mice of the vehicle group and the blank control group by tail vein; the first day of administration was designated as Day 1, administrating for 13 consecutive days. On Day 0 (before administration), Day 4, Day 7, Day 10 and Day 13 of administration, the volume (length × width² × 0.52) of subcutaneous tumors in mice was measured with a vernier caliper ^{[40-41]}

The results show that, the tumor volume of mice in the vehicle group is larger than that of mice in the plasminogen group on Days 7, 10 and 13, and the statistical difference is close to significant on Day 13 (P=0.05) (Fig. 6A). The differences of tumor volumes on Day 13 and Day 4 between the vehicle group and the plasminogen group were compared and analyzed. The results show that, the increased tumor volume of mice in the vehicle group is significantly greater than that of mice in the plasminogen group, and the statistical difference is significant (P=0.01) (Fig. 6B). The above results suggest that plasminogen can inhibit the growth of colon cancer tumors.

### Example 6: Plasminogen Improves Pain sensation of Lung Cancer Model Mice

Mouse Lewis lung cancer cells (LLC) were purchased from Shanghai Institutes for Biological Sciences, Chinese Academy of Sciences, hereinafter referred to as LLC. The LLC was placed in DMEM (GIBCO, Catalog No. 15140122) culture solution containing 10% fetal bovine serum, then culturing in a 5% CO₂ incubator at 37°C. After the growth of the cells in the culture flask reached 90% confluency, they were digested with trypsin (0.02% EDTA added to 0.05% trypsin) (Sigma, 74799), then washing 3 times with normal saline. The cells were resuspended in saline, counting under a microscope to confirm that the cells were in single suspension. Finally, the cells were resuspended with normal saline at a cell concentration of 10⁷ cells/ml, then placing on ice for later use.

Thirty C57 mice of 8-9 week-age were weighed and randomly divided into two groups, i.e., 6 mice in the blank control group, and 24 mice in the model group. After grouping, the mice were anesthetized with 2% isoflurane by using a respiratory anesthesia machine; the hair on the left leg was depilated, and a 0.5 cm shallow incision was made on the skin of the distal femoral condyle after disinfection with iodine solution; then a blunt dissection was performed on the patellar ligament to expose the distal femoral condyle with minimal damage; mice in the model group were slowly injected with 10 µl LLC cell suspension (2×10⁵ cells/µl) into the medullary cavity at the distal end of left femur by a 50 µl sterile microinjector. The mice in the control group were injected with 10 µl sterile normal saline in the bone cavity; after the injection, the syringe was placed in-situ for 90 s to allow the cells to fill the bone cavity, and the injection hole was sealed with sterile bone wax to prevent cell leakage after the syringe was removed, and then the wound was sutured with a 5.0 ligature silk thread; the mouse after the operation was placed on a heating pad to keep warm, and the mouse was put back into the cage after waking up. On Day 8 of surgical modeling, all mice were weighed and electronically detected for pain. The mice in the model group were divided into groups according to the measurement results of pain and body weight, i.e., 12 mice in the vehicle group, and 12 mice in the plasminogen group. After the grouping was completed, human plasminogen was injected into mice of the plasminogen group by tail vein at 50 mg/kg/mouse/day, and the vehicle was injected into the mice of the vehicle group and the blank control group by tail vein at 5 ml/kg/mouse/day; the first day of administration was designated as Day 1. On Day 7, Von-Frey filaments (Stoelting, USA) were used to detect the sensitivity of animals to mechanical injury. With 2.0 g force as the initial force, the left foot was firstly detected. If 4 out of 5 stimuli cause a paw withdrawal response, it is considered positive, and recorded as the animal's threshold for mechanical injury. If the 2.0 g force stimulation response is negative, its right foot will be stimulated with a higher force, if it is positive, it will be counted as its threshold, if it is negative, continuing to stimulate its left foot with a higher force, and stimulating the mice alternately until a positive reaction occurs to the right foot ^{[48]}. Von-Frey filament electronic pain measurement test is a commonly method for evaluating cancer pain in preclinical research and clinical research.

The results show that the pain threshold of mice in the blank control group is normal, and the pain threshold of mice in the vehicle group is increased. Compared with the vehicle group, the pain threshold of mice in the plasminogen group is significantly lower, and the statistical P value of the two groups is 0.003, and the pain threshold of mice in the plasminogen group is close to that of mice in the blank control group (Fig. 7). The results show that plasminogen can significantly improve the pain sensation of lung cancer model mice.

### Example 7: Plasminogen Improves General Physical Condition and Mental State of Lung Cancer Model Mice

11 female C57 mice of 6-7 week-age were randomly divided into two groups according to body weight, i.e., 5 mice in the plasminogen group, and 6 mice in the vehicle control group. Two groups of mice were intraperitoneally injected with pentobarbital sodium at 50 mg/kg body weight, and 2 × 10⁶ cells/200 µl LLC suspension solution was subcutaneously inoculated on the back of the mice after anesthesia ^{[46,47]}. The mice were administrated from the day of inoculation with the cells, and this day was recorded as Day 1; particularly, human plasminogen was injected at 1 mg/0.1 mL/mouse/day by tail vein into mice in the plasminogen group, and the same volume of vehicle PBS was injected by tail vein into mice in the vehicle control group every day, observing and taking photos to record the tumor situation and the mental state of the mice every day.

The results show that on Day 20, the mice in the vehicle PBS control group (Fig. 8A) erected their hairs, their movements were sluggish, their spirits were listless, the tumors were severely ruptured, and the tumor wounds were obviously festered and bleeding (marked by arrows); the mice in the plasminogen group (Fig. 8B) moved freely and were in a good state of mind, there was no obvious ulceration of the tumor and a small amount of scab on the epidermis of the tumor. This observation suggests that plasminogen can improve the general physical condition and mental state of lung cancer model mice.

### Example 8: Plasminogen Improves the Survival Rate of Lung Cancer Model Mice

11 female C57 mice of 6-7 week-age were randomly divided into two groups according to body weight, i.e., 5 mice in the plasminogen group, and 6 mice in the vehicle control group. Two groups of mice were intraperitoneally injected with pentobarbital sodium at 50 mg/kg body weight, and 2 × 10⁶ cells/200 µl LLC suspension solution was subcutaneously inoculated on the back of the mice after anesthesia ^{[46,47]}. The mice were administrated from the day of inoculation with the cells, and this day was recorded as Day 1; particularly, human plasminogen was injected at 1 mg/0.1mL/mouse/day by tail vein into mice in the plasminogen group, and the same volume of vehicle PBS was injected by tail vein into mice in the vehicle control group every day. The survival of the mice was observed and recorded every day for 24 consecutive days. During the observation period, the animals were administrated according to the protocol every day.

It can be seen from the survival curve that, during the 24 days of observation, the survival rate of mice in the vehicle control group is significantly lower than that of mice in the plasminogen group, and the statistical difference is significant (Fig. 9). The results show that plasminogen can improve the survival rate and prolong the survival time of the lung cancer model mice.

### Example 9: Plasminogen Inhibits the Growth of Lung Cancer

30 male C57 mice of 13-14 week-age were randomly divided into two groups according to body weight, i.e., 6 mice in the blank control group, and 24 mice in the model group. The mice in the two groups were anesthetized with 2% isoflurane. After the mice were anesthetized, the back skin at the 4-6 lumbar vertebrae was disinfected, and 1 × 10⁶ cells of LLC suspension solution were subcutaneously inoculated ^{[46,47]}. After the cell injection, the tumor growth status of the mice was carefully observed. 7 days after the tumor cell inoculation, all the mice were tested for body weight, tumor volume and open field. The mice in the model group were randomly divided into two groups according to the test results, i.e., 12 mice in each of the vehicle control group and the plasminogen group; the administration was started, and this day was recorded as Day 1. Human plasminogen was injected at 1 mg/0.1 mL/mouse/day by tail vein into mice in the plasminogen group, and the same volume of vehicle PBS was injected by tail vein into mice in the vehicle control group, administrating for 14 consecutive days. Tumor volume and body weight were measured on Days 1, 4, 8, 11, and 15. On Day 15, the mice were sacrificed, and the tumor tissues were collected and weighed. Tumor coefficient = tumor tissue weight/body weight* 100.

### Tumor Coefficient

The results show that the tumor coefficient of mice in the plasminogen group is significantly smaller than that of mice in the vehicle group, and the statistical difference is close to significant (P=0.09) (Fig. 10).

### Tumor Volume

The results show that the tumor volume of mice in the plasminogen group is significantly smaller than that of mice in the vehicle group, and the statistical difference is extremely significant on Day 4 (** indicates P<0.01) (Fig. 11).

The above results indicate that plasminogen can inhibit the growth of lung cancer cells.

### Example 10: Plasminogen Promotes Recovery of Activity Ability in Lung Cancer Model Mice

30 male C57 mice of 13-14 week-age were randomly divided into two groups according to body weight, i.e., 6 mice in the blank control group, and 24 mice in the model group. The mice in the two groups were anesthetized with 2% isoflurane. After the mice were anesthetized, the back skin at the 4-6 lumbar vertebrae was disinfected, and 1 × 10⁶ cells of LLC suspension solution were subcutaneously inoculated ^{[46,47]}. After the cell injection, the tumor growth status of the mice was carefully observed. 7 days after the tumor cell inoculation, all the mice were tested for body weight, tumor volume and open field. The mice in the model group were randomly divided into two groups according to the test results, i.e., 12 mice in each of the vehicle control group and the plasminogen group; the administration was started, and this day was recorded as Day 1. Human plasminogen was injected at 1 mg/0.1 mL/mouse/day by tail vein into mice in the plasminogen group, and the same volume of vehicle PBS was injected by tail vein into mice in the vehicle control group, administrating for 7 consecutive days. The open field test was carried out on Day 8 of administration.

### Open Field Test

During the experiment, the mice were put into the center of the bottom surface of the open field (40 × 40 × 40 cm), and the camera shooting and timing were performed at the same time. The observation lasted for 5 minutes, and each mouse was subjected to 3 experiments. The Smart system is a complete and easy-to-use video tracking system for evaluating the behavior of laboratory animals. It allows recording trajectories, activities, specific behaviors (such as rotation, stretching and feeding) and events, and performs calculations of various analysis parameters. In this experiment, the Smart3.0 system was used to record and analyze the movement of mice, and record the boundary resting time rate of mice. In each experiment, 70% alcohol was used to wipe the box to prevent olfactory bias.

The results show that the blank control mice have a certain boundary resting time rate; the boundary resting time rate of mice in the vehicle group is significantly higher than that of mice in the blank control group; the boundary resting time rate of mice in the plasminogen group is significantly less than that of mice in the vehicle group, and the statistical difference is significant (* indicates P<0.05) (Fig. 12). The results indicate that plasminogen can promote the recovery of activity and behavior in lung cancer model mice.

### Example 11: Plasminogen Inhibits Tumor Growth in Lung Cancer Model Mice

24 SCID (severe combined immunodeficiency) mice (NOD.CB17-Prkdcscid/NcrCrl, NOD SCID for short, purchased from Beijing Vital River Laboratory Animal Technology Co., Ltd., strain code 40624) of 7-10 week-age were weighed to randomly divide into two groups, i.e., 8 mice in the blank control group, and 16 mice in the model group. After grouping, all mice in the model group were anesthetized with 2% isoflurane, then disinfecting their backs, and subcutaneously inoculating a suspension solution of 1×10⁶ Lewis lung carcinoma cells-GFP cells (mouse lung cancer cells stably transfected with green fluorescent protein (GFP), referred to as LLC-GFP cells) (purchased from Shanghai Meixuan Biotechnology Co., Ltd.), and observing for 7 days. On Day 8 after tumor cell inoculation, all mice were tested for body weight, and the mice in the model group were tested for tumor volume. According to the test results, mice in the model group were randomly divided into two groups, i.e., 8 mice in the vehicle group, 8 mice in the plasminogen group; and mice in the blank control group were not treated. After grouping of the mice was completed, human plasminogen was injected at 1 mg/0.1 ml/mouse/day by tail vein into mice in the plasminogen group, the same volume of vehicle PBS was injected by tail vein into mice in the vehicle group, and the mice in the blank control group were not treated with administration, continuously administrating for 10 days. The volume of subcutaneous tumors in mice (length × width ² × 0.52) was measured with a vernier caliper on Day 0 (before administration), and Days 4, 7 and 10 of administration ^{[40-41]}.

The results show that the tumor volume of mice in the plasminogen group is significantly smaller than that of mice in the vehicle group at each measurement time point, the statistical P value is 0.22 on Day 4 of administration, 0.003 on Day 7 of administration, and 0.07 on Day 10 of administration (Fig. 13). It shows that plasminogen can significantly inhibit tumor growth in lung cancer model mice.

### Example 12: Plasminogen Improves Pain Sensation in Cancer Pain Model Mice

18 female and 18 male Balb/c mice of 8-9 week-age were randomly divided into two groups after weighing, i.e., 12 mice in the blank control group, and 24 mice in the model group. After grouping, the mice were anesthetized with 2% isoflurane by using a respiratory anesthesia machine; the hair on the left leg was depilated, and a 0.5 cm shallow incision was made on the skin of the distal femoral condyle after disinfection with iodine solution; then a blunt dissection was performed on the patellar ligament to expose the distal femoral condyle with minimal damage; mice in the model group were slowly injected with 5 µl CT-26 cell suspension solution (2×10⁴ cells/µl) into the medullary cavity at the distal end of left femur by a 50 µl sterile microinjector. The mice in the control group were injected with 5 µl sterile normal saline in the bone cavity; after the injection, the syringe was placed in-situ for 90 s to allow the cells to fill the bone cavity, and the injection hole was sealed with sterile bone wax to prevent cell leakage after the syringe was removed, and then the wound was sutured with a 5.0 ligature silk thread; the mouse after the operation was placed on a heating pad to keep warm, and the mouse was put back into the cage after waking up. On Day 8 of surgical modeling, all mice were weighed, and the mice in the model group were randomly divided into groups according to the measurement results of body weight, i.e., 12 mice in each of the vehicle group and the plasminogen group. After the grouping was completed, human plasminogen was injected into mice of the plasminogen group by tail vein at 50 mg/kg/mouse/day, and the vehicle was injected into the mice of the vehicle group and the blank control group by tail vein at 5ml/kg/mouse/day; the first day of administration was designated as Day 1, administrating for 7 consecutive days. On Day 8, the cold and hot plate pain test was performed, and the pain response of the mice was recorded. The day before the test, the temperature of the equipment was set to a constant temperature of 20°C, then placing the mice into it one by one for 5 min to adapt. On the day of testing, the hot and cold plate RAMP mode was selected, the initial temperature was set at 20°C, the final temperature was set at 4°C, and the time period from the initial temperature to the final temperature was set as 5 min. After the equipment temperature reached the initial temperature, placing the mouse to be tested on the cold plate and clicking Start button for timing and cooling down, observing the mouse until its rear foot is contracted to lift, then recording the time and the displayed temperature when the mouse's hind foot is contracted to lift, the longest detection time was 6 minutes, measuring 3 times continuously. Note that after each mouse experiment, the hot and cold plate should be cleaned and wiped with disinfectant before starting the experiment of the next mouse, thereby avoiding mutual interference.

### The Temperature When the Mouse's Hind Foot Is Contracted to Lift

The results show that the temperature when the mouse's hind foot is contracted to lift in the vehicle group is significantly higher than that in the blank control group, and the temperature when the mouse's hind foot is contracted to lift in the plasminogen group is significantly lower than that in the vehicle group and is close to that in the blank control group. The results of statistical analysis show a comparison between the female mice in the plasminogen group and the vehicle group, P=0.007; and a comparison between the male mice in the plasminogen group and the vehicle group, P=0.043 (Fig. 14A).

### The Time When the Mouse's Hind Foot Is Contracted to Lift

The results show that the time when the mouse's hind foot is contracted to lift in the vehicle group is significantly earlier than that in the blank control group, and the temperature when the mouse's hind foot is contracted to lift in the plasminogen group is significantly later than that in the vehicle group and is close to that in the blank control group. The results of statistical analysis show a comparison between the female mice in the plasminogen group and the vehicle group, P=0.004; and a comparison between the male mice in the plasminogen group and the vehicle group, P=0.023 (Fig. 14B).

The above results show that plasminogen can relieve pain sensation in cancer pain model mice.

### Example 13: Plasminogen Improves and Inhibits Pain Sensation in Cancer Pain Model Mice

Thirty C57 mice of 8-9 week-age were weighed and randomly divided into two groups, i.e., 6 mice in the blank control group, and 24 mice in the model group. After grouping, the mice were anesthetized with 2% isoflurane by using a respiratory anesthesia machine; the hair on the left leg was depilated, and a 0.5 cm shallow incision was made on the skin of the distal femoral condyle after disinfection with iodine solution; then a blunt dissection was performed on the patellar ligament to expose the distal femoral condyle with minimal damage; mice in the model group were slowly injected with 10 µl LLC cell suspension solution (2×10⁵ cells/µl) into the medullary cavity at the distal end of left femur by a 50 µl sterile microinjector. The mice in the control group were injected with 10 µl sterile normal saline in the bone cavity; after the injection, the syringe was placed in-situ for 90 s to allow the cells to fill the bone cavity, and the injection hole was sealed with sterile bone wax to prevent cell leakage after the syringe was removed, and then the wound was sutured with a 5.0 ligature silk thread; the mouse after the operation was placed on a heating pad to keep warm, and the mouse was put back into the cage after waking up. On Day 8 of surgical modeling, all mice were weighed and electronically detected for pain. The mice in the model group were randomly divided into groups according to the measurement results of body weight, i.e., 12 mice in each of the vehicle group and the plasminogen group. After the grouping was completed, human plasminogen was injected into mice of the plasminogen group by tail vein at 50 mg/kg/mouse/day, and the vehicle was injected into the mice of the vehicle group and the blank control group by tail vein at 5 ml/kg/mouse/day; the first day of administration was designated as Day 1, administrating for 20 consecutive days. On Day 20, a forceps tenderness test was performed. The specific steps were as follows: placing the animal to be tested on a fixed frame, exposing the left and right paws; clamping the mouse's hind paw with a plier, and measuring the maximum pressure on it; recording the desired measuring pressure values. The left and right hind paws of each mouse were measured 4 times.

The results show that the pain threshold of mice in the plasminogen group is significantly higher than that of mice in the vehicle group, and the statistical analysis P value is 0.006 (Fig. 15). It is suggested that plasminogen can relieve the pain sensation of cancer pain model mice.

### Example 14: Plasminogen Can Inhibit Tumor Growth in Lung Cancer Model Mice

21 SCID (severe combined immunodeficiency) mice (NOD.CB17-Prkdcscid/NcrCrl, NOD SCID for short, purchased from Beijing Vital River Laboratory Animal Technology Co., Ltd., strain code 40624) of 7-10 week-age were weighed to randomly divide into two groups, i.e., 7 mice in the blank control group, and 14 mice in the model group. After grouping, all mice in the model group were anesthetized with 2% isoflurane (purchased from Lunan Better Pharmaceutical Co., Ltd.), then disinfecting their backs, and subcutaneously inoculating a suspension solution of 1×10⁶ Lewis lung carcinoma cells-GFP cells (mouse lung cancer cells stably transfected with green fluorescent protein (GFP), referred to as LLC-GFP cells) (purchased from Shanghai Meixuan Biotechnology Co., Ltd.), and observing for 7 days. On Day 8 after tumor cell inoculation, all mice were tested for body weight, and the mice in the model group were tested for tumor volume. According to the test results, mice in the model group were randomly divided into two groups, i.e., 7 mice in the vehicle group, 7 mice in the plasminogen group; and mice in the blank control group were not treated. After grouping of the mice was completed, human plasminogen was injected at 1 mg/0.1 ml/mouse/day by tail vein into mice in the plasminogen group, the same volume of vehicle PBS was injected by tail vein into mice in the vehicle group, and the mice in the blank control group were not treated with administration, continuously administrating for 10 days. The first day of administration was designated as Day 0, and the tumor volume was measured on Days 0, 4, 7 and 10.

The results show that the tumor volume of mice in the plasminogen group is not different from that of mice in the vehicle group on Day 0, and the tumor volume in the plasminogen group is significantly smaller than that of mice in the vehicle group on Days 4, 7 and 10; and the statistical analysis P values are respectively 0.22, 0.003 and 0.07 (Fig. 16). It shows that plasminogen can significantly inhibit the growth of lung cancer cells.

### Example 15: The Therapeutic Effect of Plasminogen on Patients with Esophageal Cancer

The male patient of 72-year-old had clear consciousness, no history of diabetes and heart disease, but a history of hypertension. He was diagnosed with esophageal cancer 6 months ago, and started chemotherapy. The patient signed an informed consent form to receive human plasminogen treatment voluntarily, which was approved by the hospital ethics committee. Human plasminogen was started to be administrated after the fourth chemotherapy, and human plasminogen was also used after the fifth, sixth and seventh chemotherapy.

### The First Course of Treatment

After chemotherapy, the patient had symptoms of fatigue and nausea; the lower lip was broken in two places, and the palms of both hands were red. Other medicines were taken every day: Xuesusheng Granules, Shengxuetiaoyuan Granules, Ondansetron Hydrochloride Tablets.

Dosing regimen of human plasminogen: the administration mode was intravenous injection. 21 days of administration constituted a course of treatment, and the administration frequency was twice a day for the first 5 days, and once a day thereafter. Dosage per day: 40 mg on the first day, then gradually increased to 190 mg on Day 21.

The curative effect on the patient after administration is recorded in the form of 1-10 points of score, and the situation of the first day when there is no administration is recorded as 10. If the symptom is aggravated, the score will increase, and if the symptom is relieved, the score will decrease; and 1 is the lightest symptom.

On Day 3 of administration, the patient's condition was improved; and the overall impression score was 7 points, the mental state score is 8 points, the appetite score was 7 points, and the nausea subsiding score was 0 points. On Day 6 of administration, the patient's condition was further improved; and the overall impression score was 4 points, the mental state score was 5 points, the appetite score was 6 points, the left and right palm color scores were both 8 points, the ulceration of the lower lip was basically healed, and the hair basically did not fall out. On Day 21, the overall impression score was 1 point, the mental state score was 0 point, the appetite score was 0 point, and the left and right palm color scores were 3 and 4 points, respectively.

### The Second Course of Treatment

10 days after the first course of treatment with human plasminogen, the fifth chemotherapy was performed. After one day of chemotherapy, the body felt weak. Other medicines were taken at the same time: lansoprazole enteric-coated tablets.

Dosing regimen for the second course of treatment of human plasminogen: the administration mode was intravenous injection. The administration period was 21 days, the administration frequency was once a day, and the dosage was 100-120 mg.

Efficacy: the patient's mental state was good, appetite was good, sleep was good, the body was soft and improved, and there was no other discomfort.

### The Third Course of Treatment

The sixth chemotherapy was performed 5 days after the end of the second course of treatment. After the chemotherapy, the patient self-reported no discomfort. Other medicines were taken at the same time: thymosin enteric-coated tablets.

Dosing regimen for the third course of treatment of human plasminogen: the administration mode was intravenous injection. The administration period was 7 days, the administration frequency was once a day, and the dose was 100-115 mg.

Efficacy: The patient's condition was improved, and the overall impression score was 6 points compared with that before the administration. The results show that before administration, an esophageal tumor mass (marked by the arrow) can be seen in the esophagus at a position 20 cm-30 cm away from the incisors of the patient, involving the esophagus for 3/4 of the circumference, and the tumor mass is fragile and easy to bleed; the esophagus is narrow, and the gastroscopy body barely passes through it. Four weeks after the third course of treatment, the results of electronic gastroscopy show that a 0.8 cm-size raised lesion (marked by the arrow) can be seen in the esophagus at a position 21 cm away from the incisors, the mucosa is smooth; and a 2 cm-size ulcerative raised lesion can be seen in the esophagus at a position 22-24cm away from the incisors, involving the esophagus for 2/5 of the circumference; a 0.3 cm-size polyp can be seen at a position 27 cm away from the incisors, the mucosa is smooth; and the three lesions are not connected to each other. The volume of the esophageal cancer mass of the patient is significantly reduced after treatment with plasminogen and chemotherapy.

### REFERENCES

[1] Alexander CM and Werb, Z. (1991). Extracellular matrix degradation. In Cell Biology of Extracellular Matrix, Hay ED, ed. (New York: Plenum Press), pp. 255-302.
[2] Werb, Z., Mainardi, C.L., Vater, C.A., and Harris, E.D., Jr. (1977). Endogenous activiation of latent collagenase by rheumatoid synovial cells. Evidence for a role of plasminogen activator. N. Engl. J. Med. 296, 1017-1023.
[3] He, C.S., Wilhelm, S.M., Pentland, A.P., Marmer, B.L., Grant, G.A., Eisen, A.Z., and Goldberg, G.I. (1989). Tissue cooperation in a proteolytic cascade activating human interstitial collagenase. Proc. Natl. Acad. Sci. U. S. A 86, 2632-2636.
[4] Stoppelli, M.P., Corti, A., Soffientini, A., Cassani, G., Blasi, F., and Assoian, R.K. (1985). Differentiation-enhanced binding of the amino-terminal fragment of human urokinase plasminogen activator to a specific receptor on U937 monocytes. Proc. Natl. Acad. Sci. U. S. A 82, 4939-4943.
[5] Vassalli, J.D., Baccino, D., and Belin, D. (1985). A cellular binding site for the Mr 55,000 form of the human plasminogen activator, urokinase. J. Cell Biol. 100, 86-92.
[6] Wiman, B. and Wallen, P. (1975). Structural relationship between "glutamic acid" and "lysine" forms of human plasminogen and their interaction with the NH2-terminal activation peptide as studied by affinity chromatography. Eur. J. Biochem. 50, 489-494.
[7] Saksela, O. and Rifkin, D.B. (1988). Cell-associated plasminogen activation: regulation and physiological functions. Annu. Rev. Cell Biol. 4, 93-126.
[8] Raum, D., Marcus, D., Alper, C.A., Levey, R., Taylor, P.D., and Starzl, T.E. (1980). Synthesis of human plasminogen by the liver. Science 208, 1036-1037.
[9] Castellino FJ. Biochemistry of human plasminogen. Semin Thromb Hemost. 1984 Jan; 10(1):18-23.
[10] Sottrup-Jensen, L., Zajdel, M., Claeys, H., Petersen, T.E., and Magnusson, S. (1975). Amino-acid sequence of activation cleavage site in plasminogen: homology with "pro" part of prothrombin. Proc. Natl. Acad. Sci. U. S. A 72, 2577-2581.
[11] Collen, D. and Lijnen, H.R. (1991). Basic and clinical aspects of fibrinolysis and thrombolysis. Blood 78, 3114-3124.
[12] Alexander, C.M. and Werb, Z. (1989). Proteinases and extracellular matrix remodeling. Curr. Opin. Cell Biol. 1, 974-982.
[13] Mignatti, P. and Rifkin, D.B. (1993). Biology and biochemistry of proteinases in tumor invasion. Physiol Rev. 73, 161-195.
[14] Collen, D. (2001). Ham-Wasserman lecture: role of the plasminogen system in fibrin-homeostasis and tissue remodeling. Hematology. (Am. Soc. Hematol. Educ. Program. ) 1-9.
[15] Rifkin, D.B., Moscatelli, D., Bizik, J., Quarto, N., Blei, F., Dennis, P., Flaumenhaft, R., and Mignatti, P. (1990). Growth factor control of extracellular proteolysis. Cell Differ. Dev. 32, 313-318.
[16] Andreasen, P.A., Kjoller, L., Christensen, L., and Duffy, M.J. (1997). The urokinase-type plasminogen activator system in cancer metastasis: a review. Int. J. Cancer 72, 1-22.
[17] Rifkin, D.B., Mazzieri, R., Munger, J.S., Noguera, I., and Sung, J. (1999). Proteolytic control of growth factor availability. APMIS 107, 80-85.
[18] Marder V J, Novokhatny V. Direct fibrinolytic agents: biochemical attributes, preclinical foundation and clinical potential [J]. Journal of Thrombosis and Haemostasis, 2010, 8(3): 433-444.
[19] Hunt J A, Petteway Jr S R, Scuderi P, et al. Simplified recombinant plasmin: production and fu-nctional comparison of a novel thrombolytic molecule with plasma-derived plasmin [J]. Thromb Haemost, 2008, 100(3): 413-419.
[20] Duboscq C1, Genoud V, Parborell MF et al. Impaired clot lysis by rt-PA catalyzed mini-plasminogen activation. Thromb Res. 1997 Jun 15; 86(6):505-13.
[21] Nagai N, Demarsin E, Van Hoef B, et al. Recombinant human microplasmin: production and potential therapeutic properties [J]. Journal of Thrombosis and Haemostasis, 2003, 1(2): 307-313.
[22] Cao Y, Ji RW, Davidson D et al. Kringle domains of human angiostatin. Characterization of the anti-proliferative activity on endothelial cells. J Biol Chem. 1996 Nov 15; 271(46):29461-7.
[23] O'Reilly MS, Holmgren L, Shing Y et al. Angiostatin: a novel angiogenesis inhibitor that mediates the suppression of metastases by a Lewis lung carcinoma. Cell. 1994 Oct 21; 79(2):315-28.
[24] O'Reilly MS, Wiederschain D, Stetler SW, Folkman J, Moses MA. Regulation of angiostatin production by matrix metalloproteinase-2 in a model of concomitant resistance. J Biol Chem. 1999; 274:29568-29571.
[25] Paleari L, Brigati C, Anfosso L, Del'Eva R, Albini A, Noonan DM. Anti-angiogenesis in Search of Mechanisms: Angiostatin as a Prototype. In: Weber GF, editor. Cancer Therapy: Molecular Targets in Tumor-Host Interactions. Norfolk: Horizon Scientific Press; 2005. pp. 143-168.
[26] Gurpreet K, Aulakh, Yadu Balachandran et al. Angiostatin inhibits activation and migration of neutrophils. Cell Tissue Res. 2014 Feb; 355(2):375-96.
[27] Cao Y, Chen A, An SS et al. Kringle 5 of plasminogen is a novel inhibitor of endothelial cell growth. J Biol Chem. 1997 Sep 5; 272(36):22924-8.
[28] Cao Y, Xue L. Angiostatin. Semin Thromb Hemost. 2004 Feb;30(1):83-93. Review.
[29] Soff GA. Angiostatin and angiostatin-related proteins. Cancer Metastasis Rev. 2000; 19(1-2):97-107. Review.
[30] Judah Folkman, Ezio Merler, Charles Abernathy et al. ISOLATION OF A TUMOR FACTOR RESPONSIBLE FOR ANGIOGENESIS. J Exp Med. 1971 Jan 31; 133(2): 275-288.
[31] O'Reilly MS, Holmgren L, Shing Y et al. Angiostatin: a circulating endothelial cell inhibitor that suppresses angiogenesis and tumor growth. Cold Spring Harb Symp Quant Biol. 1994; 59:471-82.
[32] US5639725A.
[33] Kurup A, Lin C-, Murry DJ et al. Recombinant human angiostatin (rhAngiostatin) in combination with paclitaxel and carboplatin in patients with advanced non-small-cell lung cancer: a phase II study from Indiana University. Ann Oncol. 2006 Jan; 17(1):97-103. Epub 2005 Nov 9.
[34] Nussenbaum F, Herman IM. Tumor angiogenesis: insights and innovations. J Oncol. 2010; 2010:132641.
[35] Lisanne C, Hamming, Ben J et al. The clinical application of angiostatic therapy in combination with radiotherapy: past, present, future. Angiogenesis (2017) 20:217-232.
[36] O'Reilly MS, Holmgren L, Chen C et al. Angiostatin induces and sustains dormancy of human primary tumors in mice. Nat Med. 1996 Jun;2(6):689-92.
[37] KENNETH C. ROBBINS, LOUIS SUMMARIA, DAVID ELWYN et al. Further Studies on the Purification and Characterization of Human Plasminogen and Plasmin. Journal of Biological Chemistry, 1965, 240 (1):541-550.
[38] Summaria L, Spitz F, Arzadon L et al. Isolation and characterization of the affinity chromatography forms of human Glu- and Lys-plasminogens and plasmins. J Biol Chem. 1976 Jun 25; 251(12):3693-9.
[39] HAGAN JJ, ABLONDI FB, DE RENZO EC. Purification and biochemical properties of human plasminogen. J Biol Chem. 1960 Apr; 235:1005-10.
[40] Tom A. Drixler, Inne H. M. Borel Rinkes, Ewan D. Ritchie et al. Continuous Administration of Angiostatin Inhibits Accelerated Growth of Colorectal Liver Metastases after Partial Hepatectomy1. CANCER RESEARCH 60, 1761-1765, March 15, 2000.
[41] ZHANGWEI XU, HAI SHI, QI LI et al. Mouse macrophage metalloelastase generates angiostatin from plasminogen and suppresses tumor angiogenesis in murine colon cancer. ONCOLOGY REPORTS 20: 81-88, 2008.
[42] Majchrzak K, Kaspera W, Szymaś J et al. Markers of angiogenesis (CD31, CD34, rCBV) and their prognostic value in low-grade gliomas. Neurol Neurochir Pol. 2013 Jul-Aug; 47(4):325-31.
[43] Lee C, Liu A, Miranda-Ribera A, Hyun SW et al. NEU1 sialidase regulates the sialylation state of CD31 and disrupts CD31-driven capillary-like tube formation in human lung microvascular endothelia. J Biol Chem. 2014 Mar 28; 289(13):9121-35.
[44] Schlereth SL, Neuser B, Caramoy A et al. Enrichment of lymphatic vessel endothelial hyaluronan receptor 1 (LYVE1)-positive macrophages around blood vessels in the normal human sclera. Invest Ophthalmol Vis Sci. 2014 Feb 10; 55(2):865-72.
[45] Davis JM, Hyjek E, Husain AN et al. Lymphatic endothelial differentiation in pulmonary lymphangioleiomyomatosis cells. J Histochem Cytochem. 2013 Aug; 61(8):580-90.
[46] Poston GJ, Adam R, Alberts S, et al. OncoSurge: a strategy for improving resectability with curative intent in metastatic colorectal cancer. JClin Oncol 2005; 23:7125-34.
[47] Karmen A, Wroblewski F, Ladue JS (Jan 1955).Transaminase activity in human blood. The Journal of Clinical Investigation. 34 (1): 126-31.
[48] Wang CS, Chang TT, Yao WJ, Wang ST, Chou P (Apr 2012). Impact of increasing alanine aminotransferase levels within normal range on incident diabetes. Journal of the Formosan Medical Association = Taiwan Yi Zhi. 111 (4): 201-8.
[49] Moungjaroen J, Nimmannit U, Callery PS, Wang L, Azad N, Lipipun V, Chanvorachote P, Rojanasakul Y (2006). Reactive oxygen species mediate caspase activation and apoptosis induced by lipoic acid in human lung epithelial cancer cells through Bcl-2 downregulation. J Pharmacol Exp Ther 319, 1062-1069.
[50] Wang L, Chanvorachote P, Toledo D, Stehlik C, Mercer RR, Castranova V, Rojanasakul Y (2008). Peroxide is a key mediator of Bcl-2 down-regulation and apoptosis induction by cisplatinin human lung cancer cells. Mol Pharmacol 73, 119-127.
[51] Jae Kyu Ryu, Mark A. Petersen, Sara G. Murray et al. Blood coagulation protein fibrinogen promotes autoimmunity and demyelination via chemokine release and antigen presentation. NATURE COMMUNICATIONS, 2015, 6:8164.
[52] Dimitrios Davalos, Katerina Akassoglou. Fibrinogen as a key regulator of inflammation in disease. Seminars in Immunopathology, 2012. 34(1):43-62.
[53] Michael S. C'Reilly, Lars Holmgren,' Yuen Shing et al. Angiostatin: A Novel Angiogenesis Inhibitor That Mediates the Suppression of Metastases by a Lewis Lung Carcinoma. Cell, Vol. 79, 315-328, October 21, 1994.
[54] Zuo J, Wen M et al.PLGA-Der p1 Vaccine Inhibited Tumor Growth in a Murine Model of Lung Cancer, Arch Med Res.2015 Dec 16.
[55] Yoon Choi a, Young Wook Yoon a, Heung Sik Na, Sun Ho Kim, Jin MO Chung. Behavioral signs of ongoing g pain and cold allodynia in a rat model of neuropathic pain, Pain, 59 (1994) 369-376.
[56] Takehiko Maeda, Daisuke Yamada, Kohichi Kawahara, Cancer pain relief achieved by disrupting tumor-driven semaphorin 3A signaling in mice, Neuroscience Letters 632 (2016) 147-151.
[57] Li B, Vanroey M, Wang C, et al. Anti-programmed death-1 synergizes with granulocyte macrophage colony-stimulating factor--secreting tumor cell immunotherapy providing therapeutic benefit to mice with established tumors[J]. Clinical Cancer Research An Official Journal of the American Association for Cancer Research, 2009, 15(5):1623.
[58] Sabino MAC, Luger N M , Mach D B , et al. Different tumors in bone each give rise to a distinct pattern of skeletal destruction, bone cancer-related pain behaviors and neurochemical changes in the central nervous system[J]. International Journal of Cancer, 2003, 104(5):550-558.
[59] Rama Krishna Nimmakayala, Surinder K. Batra, Moorthy P. Ponnusamy. Unraveling the Journey of Cancer Stem Cells from Origin to Metastasis. Biochim Biophys Acta Rev Cancer. 2018 Nov 9; 1871(1):50-63.

## Claims

1. A method for treating a tumor, comprising administrating to a subject suffering from a tumor an effective amount of one or more compounds selected from the group consisting of: a component of plasminogen activation pathway, a compound directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of a fibrinolysis inhibitor.

2. The method according to claim 1, wherein the component of plasminogen activation pathway is selected from the group consisting of: plasminogen, recombinant human plasmin, Lys-plasminogen, Glu-plasminogen, plasmin, a variant of plasminogen and plasmin and an analog thereof comprising one or more kringle domains and protease domains of plasminogen and plasmin, mini-plasminogen, mini-plasmin, micro-plasminogen, micro-plasmin, delta-plasminogen, delta-plasmin, an activator of plasminogen, tPA and uPA.

3. The method according to claim 1 or 2, wherein the antagonist of a fibrinolysis inhibitor is PAI-1, an inhibitor of complement C1, or an inhibitor of α2 anti-plasmin or α2 macroglobulin, e.g., an antibody.

4. The method according to any one of claims 1-3, wherein the compound is plasminogen.

5. The method according to any one of claims 1-4, wherein the tumor is a cancer.

6. The method according to any one of claims 1-5, wherein the tumor is one or more selected from the group consisting of: oral cancer, esophageal cancer, gastric cancer, small intestine cancer, colon cancer, rectal cancer, lung cancer, liver cancer, hepatocellular carcinoma, pancreatic cancer, gallbladder cancer, non-small cell lung (NSCL) cancer, bronchoalveolar cell lung cancer, breast cancer, ovarian cancer, cervical cancer, fallopian tube cancer, endometrial cancer, vaginal cancer, prostate cancer, urethral cancer, penile cancer, kidney cancer, ureter cancer, renal cell cancer, renal pelvis cancer, bladder cancer, head and neck cancer, skin cancer, melanoma, mesothelioma, bone cancer, thyroid cancer, parathyroid cancer, adrenal cancer, soft tissue sarcoma, glioma, pleomorphic glioblastoma, astrocytoma, Schwann cell tumor, ependymoma, medulloblastoma, meningioma, squamous cell carcinoma, and pituitary adenoma.

7. The method according to any one of claims 1-6, wherein the plasminogen has one or more effects selected from the group consisting of: reducing tumor volume, improving general survival of a subject suffering from a tumor, delaying tumor progression, inhibiting the growth of tumor cells, improving the survival rate, prolonging the survival period of a subject suffering from a tumor, relieving cancer pain, inhibiting tumor angiogenesis, promoting tumor cell necrosis or apoptosis, promoting anti-tumor immune response, regulating the expression of tumor-associated antigens or the surface molecules of lymphocytes, reducing the damage of tissues and organs caused by cancer cells, and promoting the recovery of tumor-damaged tissue structures or functions.

8. The method according to any one of claims 1-7, wherein the plasminogen level in blood or the plasminogen level in tumor tissue or non-tumor tissue of a subject suffering from a tumor is higher than, equal to or lower than the plasminogen level in blood or the plasminogen level in corresponding non-tumor tissue of a healthy subject.

9. The method according to any one of claims 1-8, wherein the fibrin level in blood or the fibrin level in tumor tissue or non-tumor tissue of a subject suffering from a tumor is higher than, equal to or lower than the fibrin level in blood or the fibrin level in corresponding non-tumor tissue of a healthy subject.

10. The method according to any one of claims 1-9, wherein the plasminogen is administered in combination with one or more selected from the group consisting of: chemotherapy, radiotherapy, surgical therapy, cell therapy and immunotherapy.

11. The method according to any one of claims 1-10, wherein the plasminogen is selected from the group consisting of: Glu-plasminogen, Lys-plasminogen, mini-plasminogen, micro-plasminogen, delta-plasminogen, and a conservatively substituted variant thereof.

12. The method according to any one of claims 1-10, wherein the plasminogen is a plasminogen protein comprising a serine protease domain and/or a lysine binding domain.

13. The method according to any one of claims 1-10, wherein the plasminogen comprises a plasminogen protein having at least 75%, 80%, 85%, 90%, 95%, 96%, 97%, 98% or 99% sequence identity with SEQ ID NO: 14 and having proteolytic activity.

14. The method according to any one of claims 1-10, wherein the plasminogen has at least 75%, 80%, 85%, 90%, 95%, 96%, 97%, 98% or 99% sequence identity with SEQ ID NO: 2, 6, 8, 10 or 12, and still has plasminogen activity.
